# EUROPEAN PATENT APPLICATION

(11) **EP 2 314 677 A1**
(43) Date of publication of application: **27.04.2011**
(21) Application number: 09800428.6
(22) Date of filing: 23.07.2009
(51) Int. Cl.: C12N 15/00, C12M 1/00, C12N 15/09, C12Q 1/68

(54) **METHOD FOR COLLECTION OF NUCLEIC ACID FROM FECAL SAMPLE, METHOD FOR ANALYSIS OF NUCLEIC ACID, AND APPARATUS FOR TREATMENT OF FECAL SAMPLE**

(30) Priority: 23.07.2008 JP 2008189684
(71) Applicant: Olympus Corporation, Shibuya-ku Tokyo 151-0072 (JP)
(72) Inventor: NAGAOKA Tomonori, Tokyo 151-0072 (JP); TANIGAMI Yasuo, Tokyo 151-0072 (JP)
(74) Representative: Wibbelmann, Jobst
(86) International application number: PCT/JP2009/063163
(87) International publication number: WO 2010/010914

(57) **Abstract**

The present invention relates to the providing of a method for recovering nucleic acid in a stool sample at high purity without requiring a complicated procedure, a nucleic acid analysis method that uses nucleic acid recovered according to the nucleic acid recovery method, and a stool sample processing apparatus used in that method, wherein the method has: (A) a step for eluting nucleic acid amplification reaction inhibitory substances by preparing a stool sample by adding a collected stool to a solution for removing nucleic acid amplification reaction inhibitory substances that has a water-soluble organic solvent as an active ingredient thereof, and storing the stool sample for a predetermined time, (B) a step for recovering a stool-derived solid fraction by removing the solution for removing nucleic acid amplification reaction inhibitory substances from the stool sample after step (A), and (C) a step for recovering nucleic acid from the stool-derived solid fraction recovered in step (B).

## Description

### TECHNICAL FIELD

The present invention relates to a method for recovering nucleic acid from a stool sample in order to recover nucleic acids from stool samples at high purity, a nucleic acid analysis method that uses nucleic acid recovered according to the nucleic acid recovery method, and a stool sample processing apparatus.
The present application claims priority on the basis of Japanese Patent Application No. 2008-189684, filed in Japan on July 23, 2008, the contents of which are incorporated herein by reference.

### BACKGROUND ART

The number of colorectal cancer patients is currently continuing to increase rapidly each year in not only the U.S. and Europe, but in Japan as well, and is becoming one of the leading causes of cancer-related deaths. This is thought to be due to the growing proliferation of a Western style diet consisting primarily of red meat among the Japanese people. More specifically, roughly 60,000 persons are diagnosed with colorectal cancer each year in Japan, and in terms of the number of deaths by organ, colorectal cancer is ranked third after gastric cancer and lung cancer, and is predicted to continue to increase in the future. On the other hand, differing from other forms of cancer, colorectal cancer has a nearly 100% cure rate if treated soon after onset. Thus, it is of the utmost importance to include colorectal cancer in early cancer screening examinations, and research and development of testing methods for early discovery of colorectal cancer is proceeding at a rapid pace.

Methods such as barium enema examinations and colonoscopies are performed as testing methods for early discovery of colorectal cancer. Barium enema examinations consist of injecting barium into the large intestine and allowing it to adhere to the mucosal membranes of the large intestine, irradiating the intestine with X-rays to capture images of any surface irregularities, and then observing the surface. On the other hand, colonoscopy consists of observing the inside of the large intestine directly with an endoscope. Colonoscopy in particular enables high levels of sensitivity and specificity, while also offering the advantage of simultaneously allowing the excision of polyps and early forms of cancer. However, in addition to be associated with high costs, these examinations place a considerable burden on the patient while also having the problem of being accompanied by complication risks. For example, barium enemas have risks associated with X-ray exposure and intestinal obstruction. In addition, colonoscopy is an invasive procedure for the subject since the endoscope is inserted directly into the large intestine. Moreover, the endoscopic procedure requires an experienced technician and the number of facilities where this examination can be performed is limited. Consequently, these examinations are not suitable for colorectal cancer examinations targeted at asymptomatic, healthy individuals as part of routine health examinations and the like.

In recent years, fecal occult blood tests have been widely performed as a non-invasive and inexpensive method for primary screening for colorectal cancer. The fecal occult blood test is a test for the presence of hemoglobin originating in erythrocytes contained in fecal matter, and is used as a method for indirectly predicting the presence of colorectal cancer. Factors behind the widespread use of the fecal occult blood test include stool samples being able to be collected and stored at room temperature eliminating the need for refrigerators, freezers and other special storage conditions, samples being able to be collected easily at home, and the test procedure being extremely simple. However, since the fecal occult blood test has low sensitivity of only about 25%, it has the problem of a high percentage of colorectal cancer being overlooked. Moreover, it also has a low positive predictive value, with the percentage of actual colorectal cancer patients among subjects judged to be positive in the fecal occult blood test being only about 10%, thus resulting in a large number of false positives. Consequently, there is a strong need for the development of a new examination method offering higher reliability.

Attention is currently focusing on new examination methods that are suitable for routine health examinations by being non-invasive, simple and highly reliable for use in testing for the presence of cancer cells and cancer cell-derived genes in stool samples. Since these examination methods investigate the presence of cancer cells or cancer cell-derived genes directly, they are considered to be more reliable than the fecal occult blood test, which tests for the presence of blood from the digestive tract that occurs indirectly accompanying the onset of colorectal cancer.

In order to accurately detect cancer cells and the like in stool samples, it is important to efficiently recover cancer cell-derived nucleic acids from those stool samples. In particular, cancer cell-derived nucleic acids are only present in trace amounts in stool samples, and since stool samples also contain large amounts of digestive remnants and bacteria, nucleic acids are decomposed extremely easily. Consequently, in order to efficiently recover nucleic acids, and particularly nucleic acids derived from mammalian cells such as human cells, from stool samples, it is important to prevent decomposition of nucleic acids within the stool and prepare the stool sample so that it can be stored stably until the time of the actual testing procedure. An example of such a stool sample processing method consists of separating cancer cells that have dissociated from the large intestine or other constituent of the digestive tract from a collected stool sample. Separation of cancer cells from stool makes it possible to inhibit the effects of bacterial proteases, DNase, RNase and other degrading enzymes. Examples of methods that have been disclosed for separating cancer cells from stool include: (1) a method for separating cells from stool, comprising: (a) a step for cooling the stool to a temperature below its gel freezing point, and (b) a step for collecting cells from the stool while maintaining at a temperature below the gel freezing point so that the stool substantially remains completely intact (see, for example, Patent Document 1). Another example of such a method consists of: (2) dispersing the stool in a transport medium containing a protease inhibitor, mucous dissolver and bactericide at a normal ambient temperature, followed by isolating the exfoliated colonocytes (see, for example, Patent Document 2).

In addition, stool also contains substances (such as bile acids and salts thereof) that have an inhibitory action on nucleic acid amplification reactions such as a polymerase chain reaction (PCR) (see, for example, Non-Patent Document 1). For example, although the average adult stool output is about 200 to 400 g/day, bile acids are reported to be excreted in stool at the level of 200 to 650 mg/day in healthy persons, and at 10 times that level in the case of patients with ileal disorders. Namely, in the case of converting to the amount per gram, about 0.5 to 3.25 mg of bile acids are contained in the stool of healthy persons while ten times that amount is contained in the stool of patients.
On the other hand, inhibitory effects of bile acid salts on PCR have been reported to occur at a concentration of about 50 µg/mL. Thus, in the case of extracting nucleic acid from stool and amplifying by PCR and the like, it is desirable to prevent carryover of inhibitory substances such as bile acid salts in the nucleic acid amplification reaction to improve amplification efficiency.

### [Prior Art Documents]

### [Patent Documents]

[Patent Document 1] Japanese Translation of PCT Application No. H11-511982
[Patent Document 2] Japanese Translation of PCT Application No. 2004-519202

### [Non-Patent Documents]

[Non-Patent Document 1] Wilson, I.G., Applied and Environmental Microbiology, 1997, Vol. 63, pp. 3741-3751

### DISCLOSURE OF THE INVENTION

### [Problems to be Solved by the Invention]

In the method described in (1) above, cells are separated while cooling a stool sample. If this separation procedure is carried out without cooling, correct detection results cannot be obtained due to deterioration of the stool sample. Thus, in order to effective prevent deterioration of the stool sample, it is important to cool the stool sample immediately after collection. However, in the case of collecting a stool sample at home for the purpose of a routine health examination and the like, it is extremely difficult and unrealistic to cool the stool sample immediately after collection. In addition, this method also has the shortcomings of the procedure for separating cells from the stool sample being complex, thereby leading to increases in examination costs.
In the method described in (2) above, although this method does not require a cooling procedure and allows preparation and storage of stool samples at room temperature by virtue of adding a bactericide and the like, since it requires a procedure for separating exfoliated colonocytes from the stool, workability again becomes inferior resulting in the problem of high costs.

Moreover, there are no descriptions whatsoever in Patent Documents 1 and 2 or Non-Patent Document 1 regarding prevention of carryover of inhibitory substances such as bile acids and bile acid salts in the nucleic acid amplification reaction when recovering nucleic acids from stool.

An object of the present invention is to provide a method for recovering nucleic acid present in stool at high purity by reducing carryover of substances such as bile acids that inhibit a nucleic acid amplification reaction without requiring a complex procedure, an analysis method that uses nucleic acids recovered according to that method, and a stool sample processing apparatus used in this recovery method and analysis method.

### [Means for Solving the Problems]

In order to solve the above problems, the inventors of the present invention found that nucleic acid can be recovered from stool at high purity by immersing a collected stool in a solution having a water-soluble organic solvent as an active ingredient thereof for removing substances that inhibit nucleic acid amplification and storing for a predetermined time prior to a nucleic acid extraction step, thereby eluting and removing nucleic acid amplification reaction inhibitory substances contained in the stool.

Namely, the present invention is as described below.
(1) A method for recovering nucleic acid from a stool sample at high purity, comprising:
   (A) a step for eluting nucleic acid amplification reaction inhibitory substances by preparing a stool sample by adding a collected stool to a solution for removing nucleic acid amplification reaction inhibitory substances that has a water-soluble organic solvent as an active ingredient thereof, and storing the stool sample for a predetermined time,
   (B) a step for recovering a stool-derived solid fraction by removing the solution for removing nucleic acid amplification reaction inhibitory substances from the stool sample after step (A), and
   (C) a step for recovering nucleic acid from the stool-derived solid fraction recovered in step (B).

(2) The stool sample may also be prepared by immediately adding a stool collected using the method for recovering nucleic acid from a stool sample described in (1) above to the solution for removing nucleic acid amplification reaction inhibitory substances without producing a suspension.

(3) The water-soluble organic solvent used in the method for recovering nucleic acid from a stool sample described in (1) or (2) above is preferably a water-soluble alcohol and/or ketone.

(4) The concentration of the water-soluble organic solvent used in the method for recovering nucleic acid from a stool sample described in (1) to (3) above is preferably 30% or more.

(5) The water-soluble organic solvent used in the method for recovering nucleic acid from a stool sample described in (3) or (4) above preferably contains one or more members of the group consisting of ethanol, propanol and methanol as a water-soluble alcohol.

(6) The water-soluble organic solvent used in the method for recovering nucleic acid from a stool sample described in (1) above is preferably ethanol.

(7) The water-soluble organic solvent used in the method for recovering nucleic acid from a stool sample described in (3) or (4) above preferably contains acetone and/or methyl ethyl ketone as a ketone.

(8) The mixing ratio of the stool and the solution for removing nucleic acid amplification reaction inhibitory substances used in method for recovering nucleic acid from a stool sample described in any of (1) to (7) above is such that the volume of the solution for removing nucleic acid amplification reaction inhibitory substances is preferably 1 or more when based on a value of 1 for stool volume.

(9) Removal of the solution for removing nucleic acid amplification reaction inhibitory substances from the stool sample in the step (B) of the method for recovering nucleic acid from a stool sample described in any of (1) to (8) above is preferably carried out by centrifugal separation.

(10) The amount of time during which the stool sample is stored in the step (A) of the method for recovering nucleic acid from a stool sample described in any of (1) to (9) above is preferably 1 hour or more.

(11) The amount of time during which the stool sample is stored in the step (A) of the method for recovering nucleic acid from a stool sample described in any of (1) to (9) above is preferably 12 hours or more.

(12) The amount of time during which the stool sample is stored in the step (A) of the method for recovering nucleic acid from a stool sample described in any of (1) to (9) above is preferably 24 hours or more.

(13) The amount of time during which the stool sample is stored in the step (A) of the method for recovering nucleic acid from a stool sample described in any of (1) to (9) above is preferably 72 hours or more.

(14) The temperature at which the stool sample is stored in the step (A) of the method for recovering nucleic acid from a stool sample described in any of (1) to (13) above is preferably 4°C or higher.

(15) The temperature at which the stool sample is stored in the step (A) of the method for recovering nucleic acid from a stool sample described in any of (1) to (13) above is preferably 20°C or higher.

(16) The solution for removing nucleic acid amplification reaction inhibitory substances used in the method for recovering nucleic acid from a stool sample described in any of (1) to (15) above preferably contains a detergent.

(17) The solution for removing nucleic acid amplification reaction inhibitory substances used in the method for recovering nucleic acid from a stool sample described in any of (1) to (16) above preferably contains a colorant.

(18) The step (C) of the method for recovering nucleic acid from a stool sample described in any of (1) to (13) above is preferably a step for simultaneously recovering nucleic acids derived from indigenous intestinal bacteria and nucleic acids derived from a creature other than indigenous intestinal bacteria from the stool-derived solid fraction recovered in step (B).

(19) The creature other than indigenous intestinal bacteria used in the method for recovering nucleic acid from a stool sample described in any of (1) to (18) above is preferably a mammalian cell.

(20) The step (C) of the method for recovering nucleic acid from a stool sample described in any of (1) to (19) above preferably has:
(a) a step for denaturing protein in the stool-derived solid fraction recovered in step (B), and eluting nucleic acid from indigenous intestinal bacteria and a creature other than the indigenous intestinal bacteria present in the stool-derived solid fraction, and
(b) a step for recovering nucleic acid eluted in the step (a).

(21) The method for recovering nucleic acid from a stool sample described in (20) above preferably has:
(c) a step for removing protein denatured by the step (a) after the step (a) and before the step (b).

(22) The protein denaturing in step (a) of the method for recovering nucleic acid from a stool sample described in (20) or (21) above is preferably carried out using one or more members selected from the group consisting of a chaotropic salt, organic solvent and detergent.

(23) The organic solvent used in the method for recovering nucleic acid from a stool sample described in (22) above is preferably phenol.

(24) Removal of denatured protein in step (c) of the method for recovering nucleic acid from a stool sample described in any of (21) to (23) above is preferably carried out using chloroform.

(25) Recovery of nucleic acid in step (b) of the method for recovering nucleic acid from a stool sample described in any of (20) to (24) above preferably has:
(b1) a step for adsorbing nucleic acid eluted in step (a) to an inorganic support, and
(b2) a step for eluting the nucleic acid adsorbed in step (b1) from the inorganic support.

(26) In addition, the present invention is a nucleic acid recovered according to the method for recovering nucleic acid from a stool sample described in any of (1) to (25) above.

(27) In addition, the present invention is a nucleic acid analysis method in which a nucleic acid derived from mammalian cells is analyzed using nucleic acid recovered from a stool sample using the method for recovering nucleic acid from a stool sample described in any of (1) to (26) above.

(28) The mammalian cells used in the nucleic acid analysis method described in (27) above are preferably gastrointestinal cells.

(29) The mammalian cells used in the nucleic acid analysis method described in (27) above are preferably exfoliated colonocytes.

(30) The nucleic acid derived from mammalian cells analyzed in the nucleic acid analysis method described in any of (27) to (29) above is preferably a marker that indicates a neoplastic transformation.

(31) The nucleic acid derived from mammalian cells analyzed in the nucleic acid analysis method described in any of (27) to (29) above is preferably a marker that indicates an inflammatory gastrointestinal disease.

(32) The analysis in the nucleic acid analysis method described in any of (27) to (31) above is preferably at least one member selected from the group consisting of mRNA expression analysis, K-ras gene mutation analysis, and DNA methylation analysis.

(33) In addition, the present invention is a stool sample processing apparatus, comprising: a solution removal mechanism that removes a solution for removing nucleic acid Amplification reaction inhibitory substances having a water-soluble organic solvent as an active ingredient thereof from a collected stool sample that has been immersed in the solution for removing nucleic acid amplification reaction inhibitory substances and stored therein for a predetermined time.

(34) The solution removal mechanism in the stool sample processing apparatus described in (33) above preferably has a centrifugal separation mechanism, a solution suction discharge mechanism, and a waste liquid recovery unit.

(35) The solution suction discharge mechanism in the stool sample processing apparatus described in (34) above is preferably a solution suction discharge nozzle, and preferably further has a mechanism that washes the solution suction discharge nozzle.

(36) The present invention is a solution for removing nucleic acid amplification reaction inhibitory substances that is a solution used to prepare a stool sample for nucleic acid recovery, has a water-soluble organic solvent as an active ingredient thereof, and lowers nucleic acid amplification reaction inhibitory substances present in recovered nucleic acids.

(37) The water-soluble organic solvent in the solution for removing nucleic acid amplification reaction inhibitory substances described in (36) above is preferably a water-soluble alcohol and/or ketone.

(38) The concentration of the water-soluble organic solvent in the solution for removing nucleic acid amplification reaction inhibitory substances described in (37) above is preferably 30% or more.

(39) The present invention is a stool collection kit having the solution for removing nucleic acid amplification reaction inhibitory substances described in any of (36) to (38) above, and a stool collection container that contains the solution for removing nucleic acid amplification reaction inhibitory substances.

### [Effects of the Invention]

According to the method for recovering nucleic acid from a stool sample of the present invention, nucleic acid amplification reaction inhibitory substances contained in the stool can be efficiently eluted and removed, thereby enabling highly pure nucleic acids to be recovered from the stool sample. In addition, according to the present invention, since a complex procedure for separating a creature or cells thereof such as mammalian cells to be detected from a stool sample is not required, both labor and costs can be effectively reduced even in cases of processing large numbers of specimens. In particular, use of the stool sample processing apparatus of the present invention makes it possible to more easily recover nucleic acid from stool samples.
In this manner, by using the method for recovering nucleic acid from a stool sample of the present invention, and the nucleic acid analysis method that uses nucleic acids recovered according to this recovery method, nucleic acids present in stool can be analyzed with both high sensitivity and high accuracy. Thus, use of the present invention can be expected to contribute to the early detection and diagnosis of colorectal cancer and various other symptoms and diseases, observation of the course of treatment, and pathological studies and the like on other abnormal states.

### BRIEF DESCRIPTION OF THE DRAWINGS

FIG. 1 is a drawing showing an embodiment of a stool sample processing apparatus of the present invention.
FIG. 2 is a drawing showing an embodiment of a stool collection container A that can be used in a stool collection kit of the present invention.
FIG. 3 is a drawing showing an embodiment of a stool collection container B that can be used in a stool collection kit of the present invention, and an example of its method of use.
FIG. 4 is a graph showing amounts of bile acid per 100 µL of an extracted RNA solution in an Example 1.
FIG. 5 is a graph showing measurement results of an Example 2 in which sodium deoxycholate concentration is plotted on the horizontal axis and the amount of GAPDH amplification is plotted on the vertical axis.

### BEST MODE FOR CARRYING OUT THE INVENTION

In the present invention, nucleic acid amplification reaction inhibitory substances refer to substances that have an inhibitory action on a nucleic acid amplification reaction, specific examples of which include bile acid and bile acid salts. In the following descriptions, nucleic acid amplification reaction inhibitory substances are abbreviated as inhibitory substance A. Furthermore, in the present invention, a nucleic acid amplification reaction refers to an amplification reaction that is accompanied by nucleic acid elongation by DNA polymerase in the manner of PCR and the like.

The method for recovering nucleic acid from a stool sample of the present invention (to also be referred to as the "nucleic acid recovery method of the present invention") consists of mixing a collected stool in a solution for removing inhibitory substance A having a water-soluble organic solvent as an active ingredient thereof, followed by storing for a predetermined time. By storing for a predetermined time in a state in which the stool is mixed with the solution for removing inhibitory substance A, inhibitory substance A, such as a bile acid or bile acid salt, contained in the stool can be efficiently eluted and removed. As a result, carryover of inhibitory substance A to nucleic acid recovered from the stool sample can be reduced considerably, thereby making it possible to recover highly pure nucleic acid.

The removal effect on inhibitory substance A as described above by the water-soluble organic solvent, namely the effect of being able to reduce carryover of inhibitory substance A to recovered nucleic acid, is presumed to be attributable to a difference in solubilities of inhibitory substance A and nucleic acid in the water-soluble organic solvent. Namely, although inhibitory substance A such as bile acid is readily soluble in water-soluble organic solvents such as alcohol, it is poorly soluble in non-polar organic solvents. On the other hand, nucleic acids are insolubilized by the effects of salt in alcohol, and are poorly soluble in water-soluble organic solvents such as alcohol. Thus, when stool is mixed with a water-soluble organic solvent such as alcohol, inhibitory substance A is eluted into the water-soluble organic solvent, and since it can be separated from nucleic acids that are insoluble in the water-soluble organic solvent component, the amount of inhibitory substance A in the recovered nucleic acids is presumed to be reduced.

More specifically, the nucleic acid recovery method of the present invention first consists of a step (A) in which a stool sample is prepared by adding a collected stool to a solution for removing nucleic acid amplification reaction inhibitory substances (to be abbreviated as removal solution S) having a water-soluble organic solvent as an active ingredient thereof. Subsequently, the prepared stool sample is stored for a predetermined time to elute the inhibitory substance A.

The removal solution S used in the nucleic acid recovery method of the present invention has a water-soluble organic solvent as an active ingredient thereof. Although biological samples such as stool normally contain large amounts of water, the removal solution S has for an active ingredient thereof a solvent having high solubility with respect to water or a water-soluble organic solvent that can be mixed at an arbitrary ratio with water. Consequently, it can be rapidly mixed with a stool sample thereby allowing the obtaining of even higher inhibitory substance A reducing effects.

The water-soluble organic solvent in the present invention is a solvent in the form of an alcohol or ketone that has a linear structure and is a liquid in the vicinity of room temperature, such as a temperature of, for example, 15 to 40°C. Furthermore, organic acids are not included in the water-soluble organic solvent of the present invention. As a result of having a water-soluble organic solvent having a linear structure as an active ingredient, mixing with stool can be carried out more rapidly than in the case of having an organic solvent having a cyclic structure in the manner of benzene rings as an active ingredient. Since organic solvents having a cyclic structure generally separate easily from water, they are difficult to mix with stool, making it difficult to obtain a high inhibitory substance A reducing effect. Even if an organic solvent having a cyclic structure dissolved to a certain degree in water, it would be necessary to mix vigorously or heat in order to uniformly disperse stool. Furthermore, it is possible to consider preliminarily preparing a mixed solution of organic solvent and water and then mixing the stool and the mixed solution in order to facilitate mixing of the stool with an organic solvent having a cyclic structure. However, it would still be necessary to vigorously mix or heat the organic solvent having a cyclic structure and water to prepare the mixed solution, thereby making this undesirable.

The water-soluble organic solvent contained in the removal solution S is preferably a water-soluble organic solvent in which the solubility with respect to water is 12% by weight or more, more preferably that in which the solubility with respect to water is 20% by weight or more, more preferably that in which the solubility with respect to water is 90% by weight or more, and particularly preferably a water-soluble organic solvent that can be mixed with water at an arbitrary ratio. Examples include methanol, ethanol, n-propanol, 2-propanol and acetone.

There are no particular limitations on the water-soluble organic solvent contained in the removal solution S provided it is a solvent that satisfies the aforementioned conditions and has low nucleic acid solubility, but allows the inhibitory substance A to be dissolved therein. Examples of this water-soluble organic solvent include alcohols such as water-soluble alcohols in the manner of methanol, ethanol, propanol, butanol or mercaptoethanol, and ketones such as acetone or methyl ethyl ketone (having solubility with respect to water of 90% by weight or more). The propanol may be n-propanol or 2-propanol. In addition, the butanol may be 1-butanol (having solubility with respect to water of 20% by weight or more) or 2-butanol (having solubility with respect to water of 12.5% by weight).

The water-soluble organic solvent contained in the removal solution S is preferably an alcohol or ketone, and is more preferably one or more members selected from the group consisting of water-soluble alcohol, acetone and methyl ethyl ketone. These solvents have high solubility with respect to inhibitory substance A such as a bile salt, and allow the obtaining of superior inhibitory substance A reducing effects. Moreover, water-soluble alcohols are more preferable from the viewpoints of availability, handling ease, safety and the like, while ethanol, propanol and methanol are even more preferable. In particular, ethanol is particularly useful in screening examinations such as routine health examinations since it has the highest degree of safety and can be handled easily even in the home.

There are no particular limitations on the concentration of the water-soluble organic solvent in the removal solution S provided it is a concentration that enables the inhibitory substance A reducing effect to be demonstrated, and can be suitably determined in consideration of the type of water-soluble organic solvent and the like. For example, in the case of using a water-soluble alcohol or ketone as an active ingredient, the concentration of the water-soluble organic solvent in the removal solution S is preferably 30% or more. By making the concentration of the water-soluble organic solvent adequately high, the water-soluble organic solvent is able to rapidly penetrate into the stool in the case of mixing the stool with the removal solution S, thereby enabling the inhibitory substance reducing effect to be demonstrated rapidly.
Furthermore, in the present invention and description of the present application, the term "%" refers to "% by volume (vol%)" unless specifically indicated otherwise.

In the case of using a water-soluble alcohol for the active ingredient in particular, the concentration of the water-soluble organic solvent in the removal solution S is preferably 30% or more, more preferably 50% or more, even more preferably 50 to 80% and particularly preferably 50% to less than 70%. If the concentration of the water-soluble organic solvent is high, adequate inhibitory substance A reducing effects can be demonstrated while requiring only a small amount of the removal solution S even for stool having a high water content.

In addition, in the case of using acetone or methyl ethyl ketone for the active ingredient, the concentration of the water-soluble organic solvent in the removal solution S is preferably 30% or more, more preferably 60% or more and even more preferably 80% or more.

In addition, the water-soluble organic solvent used in the present invention may contain only one type of water-soluble organic solvent or may be a mixed solution of two or more types of water-soluble organic solvents. For example, the water-soluble organic solvent may be a mixed solution of two or more types of alcohols, or a mixed solution of an alcohol and another type of water-soluble organic solvent. Moreover, a mixed solution of alcohol and acetone is preferable since inhibitory substance A reducing effects and nucleic acid recovery efficiency are further improved.

Although there are no particular limitations on the volume of the removal solution S added to a collected stool, if the volume of stool is assigned a value of 1, then the mixing ratio is preferably such that the volume of the removal solution S is 1 or more based on that value of 1 for the volume of stool. In the case of placing the stool in a stool collection container containing the removal solution S, the entire circumference of the stool can be immersed in the removal solution S if the volume of the removal solution S is equal to or greater than the volume of the stool, thereby enabling the effects of the present invention to be efficiently obtained. For example, in the case the volume of the stool is equal to the volume of the removal solution S, then the stool collection container containing the removal solution S can be made to be lightweight and compact. On the other hand, by adding an amount of the removal solution S that is equal to or greater than 5 times the volume of the stool, the stool can be rapidly and effectively dispersed in the removal solution S. Moreover, decreases in the concentration of the water-soluble organic solvent caused by water contained in the stool can be inhibited by this volume. The mixing ratio of the stool and the removal solution S is preferably 1:1 to 1:20, more preferably 1:3 to 1:10 and even more preferably about 1:5 for the purpose of ensuring a proper balance of both the effects of reducing the weight of the stool collection container containing the removal solution S and improving dispersibility of the stool.

Furthermore, although there are no particular limitations on the stool supplied for use in the nucleic acid recovery method of the present invention provided it is of animal origin, it is preferably of mammalian origin, and more preferably of human origin. For example, although it is preferably a human stool collected for the purpose of a routine health examination or diagnosis and the like, it may also be a stool from livestock or wild animal. In addition, although the stool used as a sample may be that which has been stored for a fixed period of time after collection, it is preferably that immediately after collection. Moreover, although the collected stool is preferably obtained immediately after voiding, that for which time has elapsed after voiding may also be used.

Although there are no particular limitations on the amount of the stool supplied for use in the nucleic acid recovery method of the present invention, it is preferably within the range of 10 mg to 1 g. If the amount of the stool is excessively large, the collection procedure becomes difficult and the size of the collection container also increases, thereby resulting in the risk of a decrease in handling ease and the like. Conversely, in the case the amount of the stool is excessively small, since the number of exfoliated colonocytes and other mammalian cells contained in the stool become excessively low, the necessary amount of nucleic acid cannot be recovered, thereby resulting in the risk of a decrease in the target accuracy of nucleic acid analysis. In addition, since stool is heterogeneous, or in other words, numerous types and forms of components are non-uniformly present therein, the stool sample is preferably collected from a wide range of the stool at the time of stool collection to avoid the effect of localization of mammalian cells.

In addition, the removal solution S is obtained by suitably diluting the water-soluble organic solvent and adjusting to a desired concentration. Although there are no particular limitations on the solvent used for dilution, water or a buffer such as PBS is preferable. In addition, the removal solution S may also contain arbitrary components in addition to the water-soluble organic solvent provided they do not impair the inhibitory substance A reducing effect of the water-soluble organic solvent. For example, the removal solution S may contain a chaotropic salt or a detergent. The containing of a chaotropic salt or detergent makes it possible to more effectively inhibit cellular activity and enzyme activity of various lyases present in the stool.

Examples of chaotropic salts contained in the removal solution S include guanidine hydrochloride, guanidine isothiocyanate, sodium iodide, sodium perchlorate and sodium trichloroacetate. A nonionic detergent is preferable for the detergent contained in the removal solution S. Examples of such ionic detergents include Tween 80, CHAPS(3-[3-cholamidopropyl-dimethylammonio]-1-propane sulfonate), Triton X-100 and Tween 20. There are no particular limitations on the type and concentration of the chaotropic salt or detergent provided they are at a concentration that allows the obtaining of inhibitory substance A reducing effects, and can be suitably determined in consideration of the amount of stool and subsequent processing steps, analysis methods and the like.

In addition, a suitable colorant may be added to the removal solution S. Coloring the removal solution S allows the obtaining of effects such as prevention of accidental swallowing and reducing stool color. The colorant is preferably a colorant used as a food additive, and is preferably blue or green and the like. Examples of colorants include Fast Green FCF (Green No. 3), Brilliant Blue FCF (Blue No. 1) and Indigo Carmine (Blue No. 2). In addition, a plurality of colorants may be used as a mixture or a single colorant may be added alone.

By mixing the stool and the removal solution S after having added the stool to the removal solution S, the effect of reducing the amount of inhibitory substance A becomes even greater. A method that allows stool and the removal solution S to be adequately mixed and can be easily carried out by a person collecting a stool is preferable. This is because adequately dispersing the stool in the removal solution S enables the water-soluble organic solvent to adequately penetrate the stool, thereby enabling inhibitory substance A present in the stool to adequately elute into the removal solution S. Furthermore, there are no particular limitations on the method used to mix the stool and removal solution S provided it is a method that involves mixing by physical means. For example, after having placed a collected stool in a sealable container preliminarily containing the removal solution S and sealing the container, the stool and the removal solution S may be mixed by vertically inverting the container or by placing the container on an agitator such as a vortex. In addition, the stool and the removal solution S may also be mixed in the presence of mixing particles. A method that uses an agitator or a method that uses mixing particles is preferable for this mixing method since mixing can be carried out rapidly and adequately. In particular, the use of a stool collection container preliminarily containing mixing particles enables mixing to be carried out easily and adequately even in an environment such as the home where there are no special equipment.

There are no particular limitations on the mixing particles provided they are of a composition that does not impair the inhibitory substance A reducing effect of the water-soluble organic solvent, and have a particle size and specific gravity that allows the stool to be rapidly and adequately dispersed in the removal solution S by colliding with the stool. Moreover, the mixing particles may consist of particles composed of one type of material or may be particles composed of two or more types of materials. Examples of such mixing particles include particles composed of glass, ceramics, plastic, latex and metal. In addition, the mixing particles may be magnetic particles or non-magnetic particles.

The effect of reducing the amount of inhibitory substance A is demonstrated by adding stool to the removal solution S and putting into a state in which the stool is immersed in the removal solution S.
Consequently, it is no longer necessarily required to mix the stool and the removal solution S immediately after having added the stool to the removal solution S. Moreover, by immersing the stool in the removal solution S, the stool and the removal solution S are mixed by vibrations during transport in the case of transporting during storage.

In this manner, a stool sample obtained by immersing the stool in the removal solution S is stored for a predetermined time in order to elute inhibitory substance A from the stool. There are no particular limitations on the duration of storage of the stool sample provided it is an amount of time that allows the effect of reducing the amount of inhibitory substance A to be demonstrated, and can be suitably determined in consideration of the type and concentration of the water-soluble organic solvent, the mixing ratio of the stool and the removal solution S, the storage temperature and the like. In the nucleic acid recovery method of the present invention, the storage time of the stool sample is preferably 1 hour or more, more preferably 12 hours or more, even more preferably 24 hours or more and particularly preferably 72 hours or more. In addition, the storage time may also be 168 hours or more. For example, by storing the stool sample for at least one hour, inhibitory substance A can be eluted from stool to a degree that the effect of carryover of inhibitory substance A from the stool can be adequately inhibited under ordinary PCR reaction conditions.

The inhibitory substance A reducing effect of the water-soluble organic solvent is obtained to a greater degree when the temperature at which the stool sample is stored is high than when it is low. More specifically, in the present invention, the storage temperature of the stool sample in step (A) is preferably 4°C or higher and more preferably 20°C or higher. However, the storage temperature is also preferably 50°C or lower. The reason for this is that there is the risk of the concentration of the water-soluble organic solvent in the stool sample decreasing below the concentration sufficient for demonstrating the inhibitory substance (A) reducing effect due to volatilization and the like as a result of storing for a long period of time under temperature conditions of 50°C or higher.

In the nucleic acid recovery method of the present invention, the inhibitory substance (A) reduction effect can be demonstrated provided the storage temperature of the stool sample is 4°C or higher. Namely, although storage in step (A) may be carried out in an environment for which the temperature is controlled using a thermostat and the like, it may also be carried out at room temperature without requiring a special, temperature-controlled environment. In addition, it can also be carried out at a temperature at which ordinary stool collection or transport of stool samples and the like is carried out. Thus, even in the case in which, for example, a stool sample prepared in step (A) is transported in the absence of temperature control, this transport period can be used as the storage period. More specifically, in cases in which the location where a person collecting a stool prepares a stool sample and the location where nucleic acid extraction is carried out are separated by a certain distance as is the case with routine health examinations and the like, and the prepared stool sample is transported to the location where nucleic acid extraction is carried out, this transport time can be considered to be the storage time in step (A) regardless of whether or not the temperature is controlled provided the temperature during transport is within the range of 4 to 50°C.

The procedure for eluting and removing inhibitory substance (A) following nucleic acid extraction can also be carried out in order to reduce the amount of inhibitory substance (A) in the nucleic acids recovered from the stool. However, in the case of carrying out examinations on a clinical specimen, this increase in testing steps leads directly to an increase in labor costs. In contrast, in the case of using the nucleic acid recovery method of the present invention, the step for eluting and removing inhibitory substances by a person collecting a stool directly immersing the stool in the removal solution S after collecting the stool can be carried out prior to a testing step at the location of testing. Thus, the nucleic acid recovery method of the present invention can be expected to lead to a reduction in clinical laboratory testing and other costs.

In addition, nucleic acids present in a stool are extremely susceptible to decomposition as was previously described. Consequently, a stool sample is normally supplied to nucleic acid recovery and analysis steps soon after it is prepared. In addition, in the case of a large time interval between stool sample preparation and nucleic acid recovery and analysis, the stool sample is stored in a low-temperature environment by freezing or refrigerating and the like in order to inhibit the progression of nucleic acid decomposition. However, in the nucleic acid recovery method of the present invention, nucleic acid can be recovered from a stool sample extremely efficiently even in the case of having stored the stool sample for a long period of time in an environment at a comparatively high temperature such as at room temperature. This is because decomposition and the like of nucleic acids contained in the stool is prevented and nucleic acids are stably maintained as a result of mixing the stool into the water-soluble organic solvent, and efficient recovery of nucleic acids in this manner by using the water-soluble organic solvent, namely efficient recovery of nucleic acids by preventing their decomposition and maintaining the nucleic acids in a stable state, is presumed to be obtained for the reasons indicated below.
(1) Cells of biological matter (herein after referred as "creature") other than indigenous intestinal flora (herein after specified to intestinal bacteria), such as mammalian cells or viruses, having nucleic acids that are to be detected are activated by the dehydrating action of the water-soluble organic solvent component.
(2) Changes over time caused by prominent decreases in cellular activity of indigenous intestinal bacteria are inhibited.
(3) The activities of various decomposing enzymes such as proteases, DNases or RNases present in stool are reduced considerably by the protein denaturing action of the water-soluble organic solvent component.

Namely, in the nucleic acid recovery method of the present invention, a removal solution S, having a water-soluble organic solvent as an active ingredient thereof, is used to prepare a stool sample. Consequently, even in the case the prepared stool sample is stored for an adequate amount of time for eluting inhibitory substance A, decomposition of nucleic acids in the stool sample can be inhibited and the nucleic acids can be maintained in a stable state during storage of the stool sample. Thus, nucleic acids can be recovered extremely efficiently in the subsequent step (C).

Although inhibitory substance A contained in the stool is preferentially eluted into the removal solution S, nucleic acids having low solubility in the removal solution S remain in the stool-derived solid fraction. Therefore, the stool-derived solid fraction is recovered by removing the removal solution S from the stool sample in step (B) following step (A). Subsequently, in step (C), highly pure nucleic acids having a low content of inhibitory substance A can be recovered by recovering nucleic acids from the recovered stool-derived solid fraction.

The method for removing the removal solution S from the stool sample in step (B) can be suitably selected from a separation method normally used in the case of separating liquid components from solid components. There are no particular limitations on this separation method provided it does not impair nucleic acids present in the stool sample and allows the liquid component of the stool sample in the form of the removal solution S to be separated from the solid component in the form of the stool-derived solid fraction for each eluted inhibitory substance A. For example, the stool-derived solid fraction may be recovered by removing the supernatant to obtain sediment following centrifugal separation, or the stool-derived solid fraction that remains on a piece of filter paper may be recovered by filtering the stool sample by filtration method. Centrifugal separation is particularly preferable in the present invention.

There are no particular limitations on the method used to recover nucleic acid in step (C), and any method can be used provided it is a method that is normally used in the case of recovering nucleic acid from a sample. Nucleic acid recovered from the stool-derived solid fraction may be DNA, RNA or both.

Nucleic acids derived mainly from a creature other than indigenous intestinal bacteria such as mammalian cells (to be referred to as mammalian cells) and nucleic acids derived from indigenous intestinal bacteria are contained in the stool-derived solid fraction. In recovering nucleic acids from the stool-derived solid fraction, although nucleic acids derived from mammalian cells and nucleic acids derived from indigenous intestinal bacteria may be recovered separately, they are particularly preferably recovered simultaneously. Simultaneously recovering nucleic acids derived from mammalian cells and nucleic acids derived from indigenous intestinal bacteria allows nucleic acids derived from indigenous intestinal bacteria present in large amounts in stool to function as carriers. As a result, nucleic acids derived from mammalian cells present in small numbers can be recovered more efficiently than in the case of recovering nucleic acids after having preliminarily isolated the mammalian cells from the stool.

For example, after denaturing protein present in the stool-derived solid fraction, and eluting nucleic acids from mammalian cells and indigenous intestinal bacteria present in the stool-derived solid fraction, by then recovering the eluted nucleic acids in step (b), nucleic acids derived from mammalian cells and nucleic acids derived from indigenous intestinal bacteria can be simultaneously recovered from the stool-derived solid fraction.

Denaturing of protein present in the stool-derived solid fraction in step (a) can be carried out by a commonly known technique. For example, protein present in the stool-derived solid fraction can be denatured by adding a compound ordinarily used as a protein denaturing agent, such as a chaotropic salt, organic solvent or detergent, to the stool-derived solid fraction. The same compounds listed as examples of chaotropic salts and detergents added to the removal solution S can be used as chaotropic salts and detergents added to the stool-derived solid fraction in step (a). Phenol is a preferable example of an organic solvent. Phenol may be neutral or acidic. In the case of using acidic phenol, RNA can be more selectively extracted into an aqueous layer than DNA. Furthermore, in the case of adding a chaotropic salt, organic solvent or detergent to the stool-derived solid fraction in step (a), one type of compound may be added or two or more types of compounds may be added.

Protein denatured in step (a) may also be removed by providing step (c) between step (a) and step (b). The quality of recovered nucleic acids can be improved by preliminarily removing denatured protein prior to recovering nucleic acid. A commonly known technique can be used to remove protein in step (c). For example, denatured protein can be removed by precipitating the denatured protein by centrifugal separation and recovering only the supernatant. In addition, centrifugal separation may be carried out after having adding chloroform and adequately agitating and mixing with a vortex and the like to precipitate the denatured protein and recover only the resulting supernatant. As a result, denatured protein can be more completely removed than in the case of simply carrying out centrifugal separation.

Recovery of nucleic acid eluted in step (b) can be carried out using a commonly known technique such as ethanol precipitation or cesium chloride ultracentrifugation. Examples of recovery methods include the following steps (b1) and (b2). Nucleic acid eluted in step (a) is adsorbed to an organic support in step (b1). Subsequently, in step (b2), the nucleic acid adsorbed in step (b1) is eluted from the inorganic support. As a result, the nucleic acid is able to be recovered. A known inorganic support capable of adsorbing nucleic acid can be used for the inorganic support used in step (b1). In addition, there are no particular limitations on the form of this inorganic support, and may be in the form of particles or a film. Examples of this inorganic support include silica-containing particles such as silica gel, silaceous oxides, glass or diatomaceous earth, and porous films such as Nylon, polycarbonate, polyacrylate or nitrocellulose film.
A solvent normally used to elute nucleic acid from these known organic supports can be suitably used for the solvent used in step (b2) in consideration of the type of nucleic acid recovered, the subsequent nucleic acid analysis method and the like. For example, purified water is particularly preferable for this elution solvent. Furthermore, the inorganic support adsorbed with nucleic acid is preferably washed using a suitable washing buffer after step (b1) and before step (b2).

In the case the stool-derived solid fraction is prepared using the removal solution S containing a chaotropic salt or detergent at a concentration adequate for eluting nucleic acid from mammalian cells and the like, step (a) can be omitted when recovering nucleic acid from the stool-derived solid fraction in step (C).

Although a protein denaturing agent such as a chaotropic salt may be added directly to the stool-derived solid fraction recovered in step (B), the protein denaturing agent is preferably added after first suspending in a suitable elution agent. In the case of recovering DNA, a phosphate buffer or Tris buffer, for example, can be used for this elution agent. An agent in which DNase has been deactivated by high-pressure steam sterilization and the like is preferable, while an agent containing a proteinase such as proteinase K is more preferable. On the other hand, in the case of recovering RNA, a citrate buffer, for example, can be used for the elution agent. However, since RNA is a substance that is extremely susceptible to decomposition, it is preferable to use a buffer that contains an RNase inhibitor such as guanidine thiocyanate or guanidine hydrochloride. Furthermore, the stool-derived solid fraction recovered in step (B) may be preliminarily washed using a suitable buffer such as PBS (phosphate-buffered saline, pH 7.4) prior to carrying out step (C).

Depending on the subsequent analysis method used, nucleic acid can also be recovered by simply adding a suitable elution agent to the stool-derived solid fraction, mixing and eluting nucleic acid from the stool-derived solid fraction without extracting or purifying nucleic acid from the stool-derived solid fraction. For example, in the case a large number of pathogens and the like are present in a stool sample and nucleic acids derived from these pathogens are to be analyzed, only the solid component is recovered from the stool sample followed by adding and mixing an elution agent such as PBS containing a proteinase such as proteinase K. Genes and the like derived from the pathogens can then be detected by using this resulting homogeneous stool sample solution directly for nucleic acid analysis. In addition, recovery of nucleic acid from the stool-derived solid fraction can also be carried out using a commercially available kit such as a nucleic acid detection kit or virus detection kit.

In the nucleic acid recovery method of the present invention, the step for immersing a collected stool in the removal solution S having a water-soluble organic solvent as an active ingredient thereof and storing for a predetermined time can be allocated to storage or transport time. Consequently, by recovering nucleic acid from a collected stool using the nucleic acid recovery method of the present invention, nucleic acid can be retained in a stable state even in cases in which time is required from stool collection to nucleic acid analysis or in cases in which the location where the stool is collected and the location where nucleic acids are analyzed are separated by a considerable distance as is the case with screening tests such as regular health examinations. In addition, the stool sample can be stored or transported while simultaneously eluting inhibitory substance A. In addition, inhibitory substance A can be easily eluted from the stool sample without necessarily requiring special equipment such as a refrigerator or freezer or special storage temperature conditions.

Step (B) in the nucleic acid recovery method of the present invention can be carried out easily and rapidly by using a processing apparatus that includes a solution removal mechanism for removing a liquid component in the form of the removal solution S from the stool sample. Although there are no particular limitations on such a solution removal mechanism provided it is an ordinary solid-liquid separation mechanism capable of separating a solid component and a liquid component, a centrifugal separation mechanism is preferable. In addition, an apparatus provided with a solution aspiration and discharge mechanism and waste liquid recovery unit for aspirating and removing a supernatant separated by the centrifugal separation mechanism enables step (B) to be carried out automatically on a plurality of stool samples. The solution aspiration and discharge mechanism is preferably a solution aspiration and discharge nozzle that aspirates supernatant from a nozzle on the end thereof. An example of such a solution aspiration and discharge nozzle is an electronic pipette.

In the case of an apparatus provided with a solution aspiration and discharge nozzle for the elution aspiration and discharge mechanism that is further provided with a mechanism for washing the solution aspiration and discharge nozzle, since the solution aspiration and discharge nozzle can be washed for each stool sample in the case of aspirating and removing supernatant from a plurality of stool samples, entrance (contamination) of saprophytic bacteria and the like into the stool sample from the outside can be avoided. Furthermore, in the case the end of the solution aspiration and discharge nozzle is a removable tip and the like, the tip can be automatically replaced for each stool sample by providing a commonly used tip attachment and removal apparatus, thereby making it possible to avoid contamination of the stool samples even in the case of not providing a solution aspiration and discharge nozzle washing mechanism.

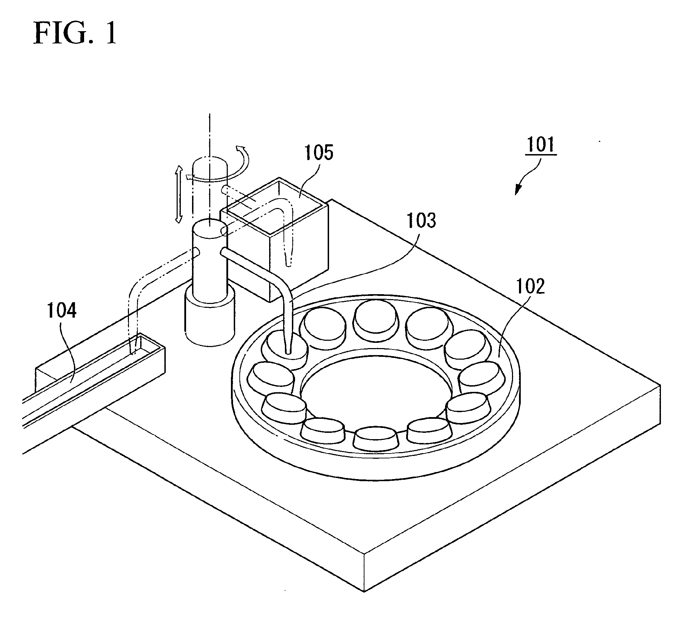

FIG. 1 is a drawing showing an embodiment of a stool sample processing apparatus for automatically carrying out step (B) in the nucleic acid recovery method of the present invention. A stool sample processing apparatus 101 in the present embodiment is provided with a centrifugal separation mechanism 102, a solution aspiration and discharge nozzle 103 for aspirating and removing a supernatant separated by the centrifugal separation mechanism 102, a waste liquid recovery unit 104, and a solution aspiration and discharge nozzle washing mechanism 105. First, a collected stool is immersed in the removal solution S in a stool collection container and stored for a predetermined time. The stool sample is placed in the centrifugal separation mechanism 102 of the stool sample processing apparatus 101 with the cover of the stool collection container open. At this time, a plurality of stool samples can be positioned and centrifuged all at once. Subsequently, the end of the solution aspiration and discharge nozzle 103 is contacted with supernatant of the centrifuged stool sample and the supernatant is removed from the stool collection container by aspirating. After discarding the supernatant aspirated by the solution aspiration and discharge nozzle 103 into the waste liquid recovery unit 104, the site of the solution aspiration and discharge nozzle that contacted the supernatant is washed by the solution aspiration and discharge nozzle washing mechanism 105. After washing, supernatant is similarly aspirated and removed from another stool sample. In this manner, step (B) can be carried out automatically by sequentially aspirating and removing supernatant from a plurality of stool samples collectively processed by centrifugal separation using the stool sample processing apparatus provided with the mechanisms as shown in FIG. 1.

According to the nucleic acid recovery method of the present invention, highly pure nucleic acids, in which carryover of inhibitory substance A such as bile acids has been significantly reduced, can be recovered efficiently. Thus, analysis of nucleic acids recovered using the nucleic acid recovery method of the present invention can be expected to allow the obtaining of highly reliable analysis results. Consequently, nucleic acids recovered according to the nucleic acid recovery method of the present invention can be used in various nucleic acid analyses. In particular, these nucleic acids are extremely preferable for analyzing not only nucleic acids derived from indigenous intestinal bacteria present in large amounts in stool, but also for analyzing nucleic acids derived from a creature other than indigenous intestinal bacteria contained only in small amounts in stool.

Furthermore, in step (C) of the nucleic acid recovery method of the present invention, in the case of simultaneously recovering nucleic acids derived from the creature other than indigenous intestinal bacteria such as mammalian cells and nucleic acids derived from indigenous intestinal bacteria, nucleic acids derived from mammalian cells, which are present in much smaller amounts than nucleic acids derived from indigenous intestinal bacteria, can be recovered extremely efficiently and at high purity. Consequently, analyzing nucleic acids by using nucleic acids recovered in this manner enables specific cancer markers for colorectal cancer and the like to be detected with high sensitivity and high accuracy.

Examples of nucleic acids derived from a creature other than indigenous intestinal bacteria include nucleic acids derived from mammalian cells such as nucleic acids derived from cancer cells, and nucleic acids derived from causative organisms of infectious diseases in the early stage or late stage of those infectious diseases such as hepatitis virus. In addition, they may also be nucleic acids derived from parasites. In particular, since the nucleic acids are recovered from stool, nucleic acids recovered according to the nucleic acid recovery method of the present invention are preferably used for analysis of nucleic acids derived from gastrointestinal tract cells such as those of the large intestine, small intestine or stomach, and are more preferably provided for analysis of nucleic acids derived from exfoliated colonocytes.

Furthermore, in the present invention, indigenous intestinal bacteria refers to bacterial cells present in comparatively large amounts in stool, and indicates indigenous bacteria that normally thrives in the intestines of animals such as humans. Examples of indigenous intestinal bacteria include obligatory anaerobic bacteria such as Bacteroides species, Eubacterium species, Bifidobacterium species or Clostridium species, and facultative anaerobic bacteria such as Escherichia species, Enterobacter species, Klebsiella species, Citrobacter species or Enterococcus species.

In addition, nucleic acids recovered according to the nucleic acid recovery method of the present invention are also preferable for nucleic acid analyses carried out for the purpose of investigating for the presence or absence of the occurrence of cancer or the onset of an infectious disease for which early discovery and high accuracy are strongly desired. In addition, they are also preferably used for nucleic acid analyses for investigating for the presence or absence of the onset of inflammatory diseases such as colitis, enteritis, gastritis or pancreatitis. They may also be used for testing for protruding lesions such as polyps as well as testing for diseases of the large intestine, small intestine, stomach, liver, gallbladder and bile duct, such as gastric ulcer.

For example, the presence or absence of the onset of colon cancer, pancreatic cancer or other cancers can be examined by detecting and analyzing nucleic acids derived from cancer cells, namely nucleic acids in which mutations and the like are occurring, by using nucleic acids recovered according to the nucleic acid recovery method of the present invention. In addition, the onset of infection or the presence of parasites can be investigated by investigating whether or not nucleic acids derived from a pathogenic organism causing the infection, such as viral nucleic acids or parasite-derived nucleic acids, are detected. In particular, testing for infections can be carried out both non-invasively and easily by using nucleic acids recovered according to the nucleic acid recovery method of the present invention to detect nucleic acids derived from pathogenic organisms excreted into the stool, such as hepatitis A virus or hepatitis E virus. In addition, presence of absence of the onset of a bacterial infection can be investigated by investigating whether or not nucleic acids derived from pathogenic bacteria other than indigenous intestinal bacteria, such as bacteria causing food poisoning or pathogenic microorganisms such as enterohemorrhagic Escherichia coli O-157, are detected.

In particular, a marker indicating a neoplastic transformation or a marker indicating an inflammatory gastrointestinal disease is preferably detected by nucleic acid analysis. Examples of markers indicating neoplastic transformation include known cancer markers such as carcinoembryonic antigen (CEA) or sialyl Tn antigen (STN), and mutations such as those of APC gene, p53 gene or K-ras gene. In addition, detection of methylation of genes such as p16, hMLH1, MGMT, p14, APC, E-cadherin, ESR1 or SFRP2 is also useful as a diagnostic marker of colon diseases (see, for example, Lind, et al., "A CpG island hypermethylation profile of primary colorectal carcinomas and colon cancer cell lines", Molecular Cancer, 2004, Vol. 3, Chapter 28). In addition, DNA derived from Helicobacter pylori present in a stool sample has been previously reported to be used as a stomach cancer marker (see, for example, Nilsson, et al., Journal of Clinical Microbiology, 2004, Vol. 42, No. 8, pp. 3781-8). On the other hand, an example of a marker that indicates an inflammatory gastrointestinal disease is nucleic acid derived from Cox-2 gene.

Nucleic acids recovered from a stool sample of the present invention can be analyzed using a known nucleic acid analysis method. Examples of nucleic acid analysis methods include methods involving detection of specific base sequence regions and methods involving quantification of nucleic acids and using PCR and the like. For example, presence of absence of the onset of cancer can be investigated by detecting the presence or absence of a base sequence region encoded by a cancer gene or by detecting the presence or absence of a genetic mutation such as a base sequence region containing a microsatellite. In the case of using DNA recovered from a stool sample, methylation of the DNA or mutations such as base insertions, deletions, substitutions, duplications or inversions, for example, can be detected. In addition, in the case of recovering RNA, cDNA can be synthesized by a reverse transcription (RT) reaction followed by using that cDNA in an amplification analysis in the same manner as DNA. In the case of using recovered RNA, mutations such as base insertions, deletions, substitutions, duplications, inversions or splicing hybrids (isoform) can be detected in the RNA. In addition, the amount of RNA expressed can also be detected. mRNA expression analyses, K-ras gene mutation analyses and DNA methylation analyses are carried out particularly preferably. Furthermore, these analyses can be carried out according to known methods in this field. In addition, a commercially available kit such as a K-ras gene mutation analysis kit or methylation detection kit may also be used.

The present invention allows the preparation of stool that is collected easily and rapidly by collecting a stool in a stool collection container preliminarily containing the removal solution S. In addition, the nucleic acid recovery method of the present invention can be carried out more easily by using a stool collection kit having the removal solution S of the present invention and a stool collection container containing the removal solution S. Furthermore, the stool collection kit may suitably have components such as a stool collection rod in addition to the removal solution S and the stool collection container containing the removal solution S.

There are no particular limitations on the shape of size and so on of the stool collection container used in the present invention, and a known stool collection container capable of containing a solvent can be used. A stool collection container in which the cover of the stool collection container and a stool collection rod are integrated into a single unit is preferable since it facilitates handling. In addition, the stool collection rod is more preferably able to collect a fixed amount of stool in order to control the amount of stool collected. An example of such a known stool collection container is the stool collection container disclosed in Japanese Examined Patent Application, Second Publication No. H6-72837.

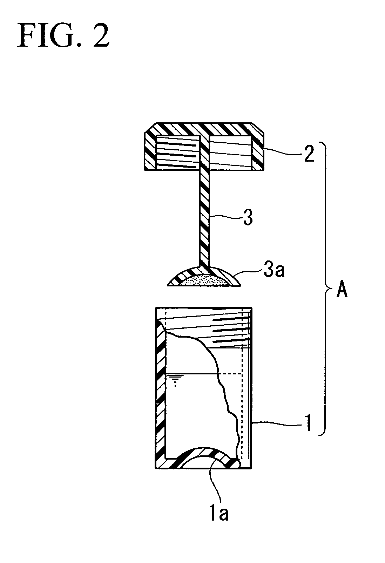

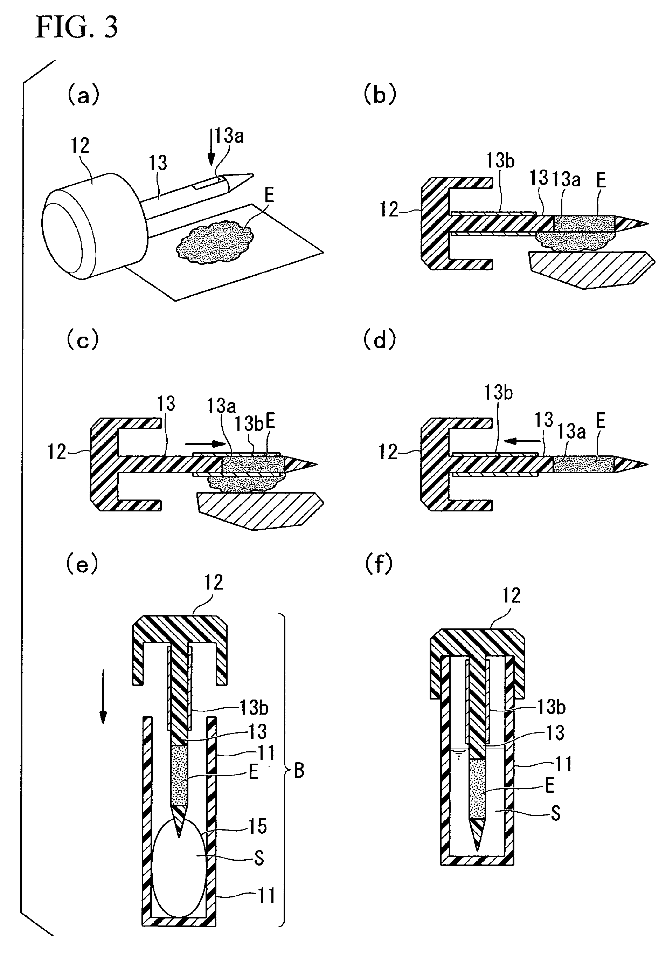

FIGS. 2 and 3 are drawings respectively showing embodiments of stool collection containers A and B able to be used in a stool collection kit of the present invention. Furthermore, stool collection containers able to be used in the stool collection kit of the present invention are not limited to these stool collection containers.

An explanation is first provided of the stool collection container of FIG. 2. The stool collection container A of the present embodiment has a cover 2 integrated with a stool collection rod 3 and a container body 1, and the removal solution S is contained within the container body 1. A cup 3a, capable of collecting a fixed amount of stool, is present on the end of the stool collection rod 3, and a net serving as a sieve is attached to the bottom of this cup 3a. On the other hand, a protrusion 1a is present on the bottom of the container body 1 that is roughly the same shape as the cup 3a and overlaps with the shape of the cup 3a. Since stool that has been collected in the cup 3a by engaging the cup 3 a with the protrusion 1a is pushed out from the net attached to the bottom of the cup 3a, stool can be rapidly dispersed in the removal solution S.

The stool collection container shown in FIG. 3 is the stool collection container B having a cover 12 integrated into a single unit with a stool collection rod 13 having a tapered end and a container body 11, and has a sealed pouch 15 within the container body 11 that contains the removal solution S. A slot 13a is formed in the distal end of the stool collection rod 13 that is capable of collecting a fixed amount of a stool E. In addition, a movable cover 13b is attached to both sides of the stool collection rod 13 that covers the slot 13a by sliding over the stool collection rod 13. The following provides an explanation of an embodiment of a usage method of the present stool collection container B.
First, as shown in FIG. 3a, the stool collection rod 13 is pressed against the stool E with the movable cover 13b moved towards the cover 12 rather than the slot 13a so that the slot 13a is completely uncovered. Next, the stool E is filled into the slot 13a as shown in FIG. 3b. While in this state, the movable cover 13b is slid towards the distal end of the stool collection rod 13 to cover the slot 13a thereby separating any excess stool E and making it possible to accurately collect the stool E in an amount equal to the size of the slot 13a (FIG. 3c). Subsequently, the movable cover 13b is returned to its original position to completely uncover the slot 13a (FIG. 3d) followed by housing the cover 12 in the container body 11 (FIG. 3e). When the stool collection rod 13 is housed in the container body 11, the tapered end of the stool collection rod 13 pierces the pouch 15 containing the removal solution S, thereby causing the container body 11 to be filled with the removal solution S to a level equal to or higher than the slot 13a containing the stool E, and enabling the stool E and the removal solution S to make direct contact (FIG. 3f). At this time, since the container body 11 is sealed by the cover 12, there is no leakage of the removal solution S. Subsequently, since the container body is moved due to transport and the like, the removal solution S and stool E within the container body 11 are mixed. Since the stool collection rod 13 is placed in a container and the solution is filled into the container only after this has been carried out, even in the case of using a removal solution S that is harmful to the body in the manner of methanol, accidents caused by leakage of solution can be avoided thereby enabling the stool collection container to be handled safely even in the home.

### [Examples]

Although the following provides a more detailed explanation of the present invention by indicating examples thereof, the present invention is not limited to the following examples. Furthermore, unless specifically indicated otherwise, the term "%" refers to "percent by volume (vol%)". In addition, MKN45 cells and SW1116 cells were used after culturing in accordance with ordinary methods.

### [Example 1]

The amount of bile acid present in an RNA solution recovered from stool according to the nucleic acid recovery method of the present invention was measured.
1 g aliquots of stool collected from a healthy volunteer were dispensed into seven 15 mL polypropylene tubes. An RNA extraction procedure was carried out on the contents of one of the tubes without adding ethanol after dispensing. More specifically, after adding and mixing a phenol mixture known as "Trizol" (Invitrogen), chloroform was added and mixed followed by centrifugal separation. The supernatant (aqueous layer) was then separated by the centrifugal separation. Sodium acetate and ethanol were then added to the separated supernatant and stirred. Subsequently, this solution was centrifuged and the ethanol supernatant was removed to obtain a precipitate. This precipitate was then air-dried at room temperature and dissolved in DEPC-treated water to obtain 100 µL of an RNA solution (1A).
After adding 10 mL of 99.5% ethanol solution (removal solution S) to the remaining six stool samples immediately after dispensing, the stool samples were stored by allowing to stand undisturbed for 12 minutes (1B), 1 hour (1C), 12 hours (1D), 24 hours (1E), 72 hours (1F) or 168 hours (1G) at 20°C. After each of the storage periods had elapsed, the samples were immediately centrifuged and the supernatant in the form of ethanol (removal solution S) was removed to obtain stool-derived solid fractions. Subsequently, an RNA extraction procedure was carried out in the same manner as (1A) on the resulting stool-derived solid fractions to obtain 100 µL aliquots of each RNA solution.
Heptadecanoic acid (C17:0) and 23 nor-deoxychoric acid (23N-DCA) were added as internal standards to each of the resulting RNA solutions followed by extraction with ether. Next, the carboxyl groups of bile acids were converted to butyl esters by butylation. In addition, the hydroxyl groups of bile acids were converted to acetyl esters by acetylation to form butyl-acetyl derivatives. The formed butyl-acetyl derivatives were analyzed by gas chromatography using OV-1701 (GL Science) with a GCMS-QP5050 System (Shimadzu) to measure the amounts of bile acid remaining in the RNA solutions.

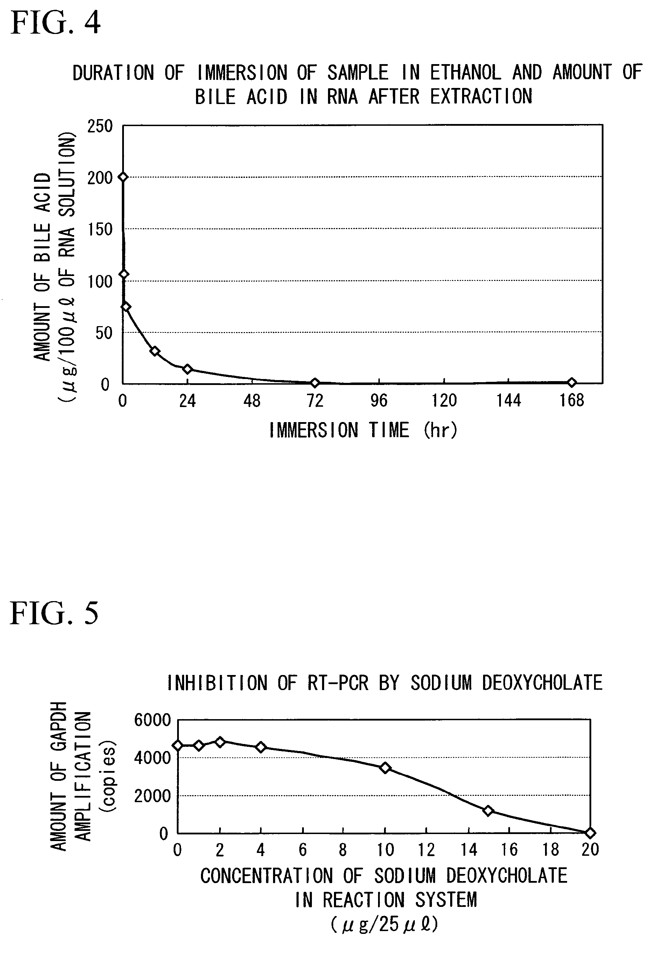

FIG. 4 is a graph showing the amounts of bile acids per 100 µL of extracted RNA solution obtained as a result of measurement. According to these results, inhibitory substance A in the form of bile acid was able to be adequately removed by immersing the stool samples in the removal solution S for a period of time of 1 hour or more. Moreover, bile acids were able to be rapidly removed during the period of time from 1 to 24 hours. In addition, there were hardly any changes observed in removal efficiency in samples for which the duration of immersion of the stool samples in the removal solution S exceeded 72 hours.

### [Example 2]

Inhibition of RT-PCR reactions by bile acid was confirmed. More specifically, a series of dilutions of sodium deoxycholate, which is a main component of bile acid, was added to a one-step real-time RT-PCR reaction solution for amplifying GAPDH (glyceraldehyde-3-phosphate dehydrogenase) using RNA recovered from cultured MKN45 cells as a template to confirm the conditions of inhibition by sodium deoxycholate.
Each reagent was mixed while observing the protocol of the One Step PrimeScript RT-PCR Kit (Takara), and the reagents were added to the reaction solutions so that the concentrations of sodium deoxycholate were 0, 1, 2, 4, 10, 15 or 20 µg/25 µL, followed by carrying out reverse transcription reactions for 5 minutes at 42°C and then for 10 seconds at 95°C. Subsequently, PCR was carried out by repeating 40 cycles of reaction conditions consisting of 5 seconds at 95°C and 30 seconds at 60°C. At that time, a TaqMan primer and probe were used for GAPDH detection of TaqMan Gene Expression Assays, Inventoried.
The prepared reagents were analyzed with the ABI Prism 7700 Sequence Detection System (Applied Biosystems) to confirm amplification efficiency in TaqMan PCR. Inhibition was evaluated by calculating the number of copies from CT values indicating known concentrations of plasmid DNA, and carrying out RT-PCR to a degree to which estimated values of the initial amount of nucleic acid obtained from these calculations decrease.
FIG. 5 is a graph showing the measurement results by plotting sodium deoxycholate concentration on the horizontal axis and the amount of GAPDH amplification on the vertical axis. On the basis of these results, inhibitory substance A was confirmed to inhibit the nucleic acid amplification reaction in cases in which sodium deoxycholate was contained in the reaction system at a concentration of 4 µg/25 µL or more.
Here, RT-PCR was carried out in a 25 µl reaction system using 5 µL of RNA obtained under the conditions of Example 1 as template. In this case, in the case of using RNA (1C) having a storage time of 1 hour, the concentration of bile acid in the reaction solution was about 3.75 µg/25 µL, while in the case of using RNA (1D) having a storage time of 12 hours, the concentration of bile acid in the reaction solution was about 1.6 µg/25 µL. In other words, the effect of bile acid introduced from stool at the time of nucleic acid recovery on the nucleic acid amplification reaction was clearly demonstrated to be effectively suppressed provided the storage time was 1 hour or more. In addition, since effects of other contaminants are also possible in the case of nucleic acid recovered from stool, it is possible that inhibition can occur due an amount of bile acid less than the concentration when stored for 12 hours (about 1.6 µg/25 µL). Consequently, it was determined that a storage time of 12 hours or more is more preferable.

### [Example 3]

1 g aliquots of stool collected from a healthy volunteer were dispensed into three 15 mL polypropylene tubes. 10 mL of a 70% ethanol solution (removal solution S) were added to one of the tubes immediately after dispensing followed by allowing to stand undisturbed for 1 minute at room temperature (20°C) to obtain a stool sample (3A). 10 mL of a 70% ethanol solution (removal solution S) were added to another tube after dispensing followed by adequately dispersing the stool and storing by allowing to stand undisturbed for 18 hours at room temperature to obtain a stool sample (3B). 10 mL of a 70% ethanol solution (removal solution S) were added to the remaining tube after dispensing followed by adequately dispersing the stool and storing by allowing to stand for 36 hours at room temperature to obtain a stool sample (3C). After allowing the storage times of each sample to elapse, the samples were immediately centrifuged and the supernatant in the form of ethanol (removal solution S) was removed to obtain stool-derived solid fractions.
In addition, RNA was extracted from each of the stool samples following removal of ethanol at the appropriate times. More specifically, after adding and mixing a phenol mixture known as "Trizol" (Invitrogen) to each of the stool-derived solid fractions, chloroform was added and mixed followed by centrifugal separation. A supernatant (aqueous phase) was obtained as a result of this centrifugal separation. Sodium acetate and ethanol were added to the supernatant and stirred followed by centrifugal separation. After obtaining a precipitate from this centrifugal separation, the precipitate was air-dried. Each precipitate was dissolved in DEPC-treated water to obtain RNA solutions.
Using a portion (5 µL) of each of the RNA solutions, cDNA was synthesized using a reverse transcription reaction kit in the form of a ReverTra Ace qPCR RT Kit. Using this cDNA as template, 12.5 µL of 2×TaqMan PCR Master Mix (Perkin-Elmer Applied Biosystems) were added followed by the addition of a human GAPDH forward primer (SEQ ID NO. 1: 5'-GAAGGTGAAGGTCGGAGTC-3') and human GAPDH reverse primer (SEQ ID NO. 2: 5'-GAAGATGGTGATGGGATTTC-3') to respective final concentrations in the reaction solution of 900 nmol to prepare a PCR solution having a final volume of 25 µL. PCR analysis using SYBR Green and the ABI Prism 7700 (Perkin-Elmer Applied Biosystems) was then carried out on this PCR solution. PCR was carried out using a thermal cycle under the conditions of a denaturation cycle of 10 seconds at 95°C followed by 45 cycles consisting of 30 seconds at 95°C, 30 seconds at 55°C and 30 seconds at 72°C. Quantification was carried out based on the results of fluorescence intensity obtained by using a dilution series of known concentrations of a standard plasmid as template.
In the case of using RNA derived from sample (3A) as template, a decrease in amplification efficiency of about 80% or more was observed in comparison with sample (3B). In addition, the difference between sample (3B) and sample (3C) was about 10%, and a difference was not observed to the degree of that between sample (1A) and sample (1B).
Namely, on the basis of these results, nucleic acid obtained using the nucleic acid recovery method of the present invention, consisting of adding the removal solution S to a stool, immersing the stool therein and then storing for a fixed period of time, was determined to yield favorable nucleic acid amplification efficiency. This is because the amount of inhibitory substance A derived from stool present in the recovered nucleic acid was able to be reduced by the nucleic acid recovery method of the present invention. Furthermore, although the identity of substances eluted and removed by the removal solution S were not confirmed in the present example, since the amplification efficiency of the recovered nucleic acid was favorable, inhibitory substance A other than bile acid contained in stool is presumed to have been eluted and removed.

### [Example 4]

1 g aliquots of stool collected from a healthy volunteer were dispensed into three 15 mL polypropylene tubes. 10 mL of a 70% ethanol solution (removal solution S) were added to one of the tubes immediately after dispensing followed by allowing to stand undisturbed for 1 minute at 4°C to obtain stool sample (4A). 10 mL of a 70% ethanol solution (removal solution S) were added to another tube after dispensing followed by adequately dispersing the stool and storing by allowing to stand undisturbed for 12 hours at 4°C to obtain stool sample (4B). 10 mL of a 70% ethanol solution (removal solution S) were added to the remaining tube after dispensing followed by adequately dispersing the stool and storing by allowing to stand for 72 hours at 4°C to obtain stool sample (4C). After allowing the storage times of each sample to elapse, the samples were centrifuged at appropriate times, and the ethanol was removed by centrifugation followed by washing using 7 mL of PBS and removing the supernatant PBS by centrifugation.
RNA was extracted and a reverse transcription reaction was carried out in the same manner as Example 3. Next, Escherichia coli gene was detected by real-time PCR with a bacterial 16S rRNA gene forward primer (SEQ ID NO. 3: 5'-AGGAGGTGATCCAACCGCA-3') and a bacterial 16S rRNA gene reverse primer (SEQ ID NO. 4: 5'-AACTGGAGGAAGGTGGGGAT-3'). In the case of using RNA derived from the sample (4A) for the template, a decrease in amplification efficiency of 70% or more was observed in comparison with sample (4B). In addition, the difference in amplification efficiency between sample (4B) and sample (4C) was about 30%.
Namely, on the basis of these results, nucleic acid obtained using the nucleic acid recovery method of the present invention demonstrated favorable nucleic acid amplification efficiency even in the case of a storage temperature of 4°C, or in other words, bile acid salts and other examples of inhibitory substance A were presumed to have been removed from the recovered nucleic acid.

### [Example 5]

An alcohol solution containing 55% methanol and 5% isopropanol was prepared instead of the ethanol used in Example 1. Alcohol solution-immersed stool samples were prepared using this alcohol solution as removal solution S, and samples were produced in the same manner as Example 1 with the exception of changing the immersion (storage) times. Namely, 10 mL of the alcohol solution were added to one of the tubes immediately after dispensing followed by adequately dispersing the stool and allowing to stand undisturbed for 1 minute at room temperature (20°C) to obtain stool sample (5A). 10 mL of the alcohol solution were added to another tube after dispensing followed by adequately dispersing the stool and storing by allowing to stand for 12 hours at room temperature to obtain stool sample (5B). 10 ml of the alcohol solution were then added to the final tube after dispensing followed by adequately dispersing the stool and storing by allowing to stand for 36 hours at room temperature to obtain stool sample (5C). The alcohol solution was removed from the samples by centrifuging at appropriate times to obtain stool-derived solid fractions. Continuing, the stool-derived solid fractions were washed with 7 mL of 99.5% ethanol, the supernatant 99.5% ethanol was removed by centrifugation, and the stool-derived solid fractions were air-dried. Next, RNA was extracted with "Trizol" and chloroform. Ethanol was added to the resulting aqueous layer and after stirring well with a vortex, a mixture of the aqueous layer and ethanol were added to an RNA recovery column (silica column) of a RNeasy Midi Kit (Qiagen), followed by centrifugation, and carrying out the RNA recovery column washing procedure and RNA elution procedure in accordance with the protocol provided to recover RNA.
Using a portion of each of the recovered RNA solutions, PCR analysis was carried out using SYBR Green in the same manner as Example 3. As a result, in the case of using RNA derived from sample (5A) for the template, a decrease in amplification efficiency of about 40% was observed in comparison with sample (5B). In addition, the difference in amplification efficiency between sample (5B) and sample (5C) was about 20%, which was about half the difference between sample (5A) and sample (5B).
Namely, on the basis of these results, even in the case of using an alcohol other than ethanol for removal solution S, inhibitory substance A such as bile acid salts was able to be extracted and removed, and highly pure nucleic acid was clearly determined to be able to be recovered. In addition, substances such as sugars and nucleic acid decomposition products are removed by first extracting RNA with an organic solvent followed by further purifying with a silica column. As a result, the amplification efficiency of nucleic acid from stool increased.

### [Example 6]

A mixture of 5.0 x 10⁵ cultured SW1116 cells derived from human colorectal cancer, in which K-ras gene codon 12 had mutated to GCT, in 4 g of stool of a healthy volunteer was used as a simulated stool of a colorectal cancer patient. 0.5 g aliquots of this stool were dispensed into six 15 mL polypropylene tubes. 10 mL of denatured ethanol (alcohol solution containing 55% ethanol and 5% isopropanol) were added to two of the tubes immediately after dispensing followed by adequately dispersing the stool and allowing to stand undisturbed for 1 minute at room temperature (20°C) to obtain stool sample (6A). 10 mL of denatured ethanol (removal solution S) were added to two other tubes after dispensing followed by adequately dispersing the stool and storing by allowing to stand undisturbed for 12 hours at room temperature to obtain stool sample (6B). 10 mL of denatured ethanol (removal solution S) were added to the remaining two tubes after dispensing followed by adequately dispersing the stool and storing by allowing to stand undisturbed for 36 hours at room temperature to obtain stool sample (6C).
After allowing each of the storage times to elapse, the denatured ethanol was removed from each of the samples (6A), (6B) and (6C) by centrifugal separation at appropriate times to obtain stool-derived solid fractions. Continuing, the stool-derived solid fractions were washed with 7 mL of 99.5% ethanol followed by removal of the supernatant 99.5% ethanol by centrifugation and air-drying the solid fractions.
In addition, DNA solutions were recovered from each of the stool-derived solid fractions from which the ethanol had been removed at appropriate times. Namely, DNA was recovered from each of the stool-derived solid fractions using a DNA extraction kit for extraction from stool in the form of the "QIAamp DNA Stool Mini Kit" (Qiagen). As a result of quantifying the concentrations of the recovered DNA by absorption photometry, nearly equal amounts of DNA were able to be recovered from each stool-derived solid fraction.
The GCT mutation sequence of K-ras codon 12 was detected by allele-specific PCR using the recovered DNA. Namely, a GCT mutant allele was amplified by designing the 3'-terminal of the sense chain primer to match the GCT mutation sequence. Furthermore, the antisense strand primer was derived from the base sequence of the intron portion. PCR was then carried out using 50 pmol each of forward primer (SEQ ID NO. 5: 5'-ACTTGTGGTAGTTGGAGCTGC-3') and reverse primer (SEQ ID NO. 6: 5'-CTCATGAAAATGGTCAGAGAAACC-3') corresponding to the K-ras codon 12 GCT mutant codon, 1.25 U of Takara Ex Taq (Takara), 1 x Taq Buffer, 200 µM dNTP Mixture and 10 ng of template nucleic acid per reaction. Temperature conditions consisted of 35 cycles of 1 minute at 94°C, 1 minute at 55°C and 1 minute at 72°C.
Following the PCR reaction, when the amplification product was confirmed by agarose electrophoresis prepared with 3% NuSieve (FMC), a 179 bp amplification product was confirmed. As a result, in the case of using DNA derived from sample (6A) for the template, amplification was not observed for one of the two samples. In addition, in the case of the other sample as well, the band was extremely light in comparison with DNA derived from sample (6B), and a decrease in amplification efficiency was observed. In addition, DNA derived from the two samples (4B) and DNA derived from the two samples (6C) both demonstrated favorable amplification, and there were hardly any differences observed in band intensity.
On the basis of the above results, nucleic acid amplification efficiency was improved the longer the storage time, or in other words, the removal efficiency of bile acid salts and the like that inhibit nucleic acid amplification was presumed to be favorable.
As a result of using DNA recovered according to the nucleic acid recovery method of the present invention, nucleic acid was clearly determined to be able to be analyzed accurately even in nucleic acid analyses requiring high accuracy such as in the case of gene mutations. In addition, although denatured alcohol consisting of a mixture of isopropanol and ethanol was used for the removal solution S in the present example, the results obtained were similar to the case of having used a 50% ethanol solution having an equal alcohol concentration.

### [Example 7]

A stool sample was prepared and nucleic acid was recovered using the stool collection container A shown in FIG. 2. This stool collection container A had the cover 2 integrated into a single unit with the stool collection rod 3, and the container body 1, and contained 5 mL of a 59% ethanol solution inside as removal solution S. In addition, the cup 3a, having a volume of 0.5 mL and four holes having a diameter of 3 mm, was provided in the distal end of the stool collection rod 3.
First, roughly 0.5 g of stool were collected into the cup 3a using the stool collection rod 3 and then placed in the stool collection container A and covered with the cover 2. As a result, the protrusion 1a in the bottom of the container body 1 pushed out stool from the holes in the cup 3a enabling stool in the form of threads to be dispersed in the solution. The stool sample prepared in this manner was designated as stool sample (7A).
On the other hand, about 0.5 g of stool were collected in a 15 mL polypropylene tube containing 5 mL of a 59% ethanol solution so that the volume ratio of stool and removal solution S was 1:10 in the same manner as the stool sample (7A), and the resulting stool sample was designated as control sample (7B).
After storing the stool sample (7A) and the control sample (7B) for 2 days at 30°C, RNA was extracted in the same manner as Example 2. At this time, the ethanol removed from the stool sample (7A) demonstrated a darker brown color than the ethanol removed from the control sample (7B). In addition, after recovering RNA and producing cDNA in the same manner as Example 3, quantitative PCR was carried out on human GAPDH gene. In the case of using RNA derived from sample (7B) as template, a decrease in amplification efficiency of about 40% was observed in comparison with RNA derived from sample (7A).
This is presumed to be the result of the use of the stool sample collection container A shown in FIG. 2 enabling the stool to be rapidly dispersed in the removal solution S thereby allowing nucleic acid to be recovered with higher purity. In addition, since the use of this stool collection container A enables preparation and storage of the stool sample to be carried out easily and immediately after stool collection by a person collecting the stool, a portion of the labor costs of operators in the testing process can be reduced.

### [Example 8]

A stool sample was prepared and nucleic acid was recovered using the stool collection container B shown in FIG. 3. The end of the stool collection rod 13 of this stool collection container was tapered, and it had the slot 13a having a volume of 2.5 mL. In addition, the stool collection container B had the cover 12 integrated into a single unit with the stool collection rod 13, and the container body 11, and the container body 11 had the sealed pouch 15 that contained a 59% methanol solution for the removal solution S.
First, about 0.1 g of stool was collected according to the procedure of FIG. 3 using this stool collection container B that contained 5 mL of the 59% methanol solution in the pouch 15, and a stool sample (8A) was prepared by mixing 2.5 mL of the 59% methanol solution with the stool in the pouch 15. After storing this stool collection container (8A) by allowing to stand undisturbed for 36 hours at 18°C, RNA was recovered in the same manner as Example 5, and when PCR analysis was carried out using SYBR Green, amplification efficiency was confirmed to be favorable.
As is clear from these results, by carrying out the nucleic acid recovery method of the present invention using the stool collection container B shown in FIG. 3, reduction in weight and size as well as safety of a stool collection can be ensured since nucleic acid can be recovered with high purity.

### [Example 9]

Nucleic acid was recovered from stool samples by carrying out the step (B) in the nucleic acid recovery method of the present invention automatically using a stool sample processing apparatus as shown in FIG. 1.
First, two stool samples were prepared in which stool and a 59% ethanol solution (removal solution S) were mixed in the stool collection container A shown in FIG. 2 in the same manner as Example 7. After storing these two stool samples for 12 hours at room temperature, the covers of the stool collection containers were opened and the stool collection containers were placed in a centrifugal separation mechanism within the stool sample processing apparatus. The stool samples were then separated into a supernatant consisting of the ethanol solution (removal solution S) and a stool-derived solid fraction by carrying out a centrifugal separation step. The supernatant of one of the stool samples was aspirated with a solution suction discharge nozzle and discarded into a waste liquid recovery unit. After discarding, the nozzle was washed with a washing layer and removal of supernatant of the remaining stool sample was carried out in the same manner.
After removing the supernatant, RNA was able to be recovered from the resulting stool-derived solid fraction in the same manner as Example 5.

### [Example 10]

1 g aliquots of stool collected from a healthy volunteer were dispensed into each of eight 15 mL polypropylene tubes. 6 mL of a 70% ethanol solution (removal solution S) were added immediately after dispensing followed by adequately dispersing the stool to prepare stool samples, after which the prepared stool samples were stored by allowing to stand for 24 hours under conditions of -4°C (10A), 0°C (10B), 4°C (10C), 8°C (10D), 12°C (10E), 16°C (10F), 20°C (10G) or 24°C (10H).
After allowing the storage times of each sample to elapse, the samples were centrifuged and the supernatant in the form of ethanol was removed to obtain stool-derived solid fractions.
Next, RNA was extracted from the resulting stool-derived solid fractions in the same manner as Example 1. PCR analysis using SYBR Green was carried out in the same manner as Example 3 using a portion of each of the recovered RNA solutions.
The analysis results for each stool sample (sample) that reacted with SYBR Green are shown in Table 1. Amplification was not confirmed in the case of using RNA derived from sample (10A) or sample (10B) as template. On the other hand, amplification was confirmed in the case of using RNA derived from samples (10C) to (10F) as template. In addition, RNA derived from sample (10G) and sample (10H) was observed to demonstrate amplification roughly double that of RNA derived from samples (10C) to (10F).
Namely, inhibitory substance A was efficiently removed at storage temperatures of 4°C and above, and was more efficiently removed at storage temperatures of 20°C and above resulting in increased nucleic acid amplification efficiency.

**[Table 1]**

| Stool Sample | 10A | 10B | 10C | 10D | 10E | 10F | 10G | 10H |
|---|---|---|---|---|---|---|---|---|
| Storage temperature | -4°C | 0°C | 4°C | 8°C | 12°C | 16°C | 20°C | 24°C |
| Amount of nucleic acid amplification | - | - | + | + | + | + | ++ | ++ |

### [Example 11],

1 g aliquots of stool collected from a healthy volunteer were dispensed into each of nine 15 mL polypropylene tubes. 6 mL of a 70% ethanol solution (removal solution S) were added immediately after dispensing followed by adequately dispersing the stool to prepare stool samples, after which the prepared stool samples were stored by allowing to stand for 24 hours under conditions of storage times of 1 minute (11A), 10 minutes (11B), 1 hour (11C), 12 hours (11D), 24 hours (11E), 36 hours (11F), 48 hours (11G), 72 hours (11H) or 168 hours (11I). Furthermore, the storage temperature was fixed at 20°C.
After allowing the storage times of each sample to elapse, RNA was recovered in the same manner as Example 10 followed by carrying out quantitative PCR analysis. The analysis results are shown in Table 2.
Although amplification was not confirmed in the case of using RNA derived from sample (11A) or sample (11B) as template, amplification was confirmed in the case of using RNA derived from sample (11C) as template. In addition, amplification roughly double that of RNA derived from sample (11 C) was observed in the case of RNA derived from sample (11D). Amplification roughly triple that of RNA derived from sample (11C) was observed in the case of samples (11E), (11F) and (11G), while amplification roughly 1.5 times that of RNA derived from sample (11D) was observed in the case of samples (11H) and (11I).
Namely, inhibitory substance A was efficiently removed in the case of storage times of 1 hour or more, even more efficiently removed at storage times of 24 hours or more, even more efficiently removed at storage times of 72 hours or more, and there were no decreases in nucleic acid amplification efficiency even at a storage time of 168 hours.

**[Table 2]**

| Stool Sample | 11A | 11B | 11C | 11D | 11E | 11F | 11G | 11H | 11I |
|---|---|---|---|---|---|---|---|---|---|
| Storage Time | 1 min | 10 min | 1 hr | 12 hr | 24 hr | 36 hr | 48 hr | 72 hr | 168 hr |
| Amount of nucleic acid amplification | - | - | + | ++ | +++ | +++ | +++ | ++++ | ++++ |

### [Reference Example 1]

1 g aliquots of stool collected from a healthy volunteer were dispensed into three 15 mL polypropylene tubes. Freezing treatment was promptly carried out on one of the tubes using liquid nitrogen immediately after dispensing to obtain a stool sample (1). 10 mL of a 70% ethanol solution were added to another tube after dispensing followed by adequately dispersing the stool and allowing to stand for 1 hour at room temperature to obtain a stool sample (2). The remaining tube was transferred to an extraction step after dispensing without adding solvent and the like to obtain a stool sample (3).
Subsequently, RNA was extracted from each stool sample. More specifically, 3 mL of a phenol mixture known as "Trizol" (Invitrogen) were added to each stool sample and adequately mixed for 30 seconds or more with a homogenizer. Subsequently, after adding 3 mL of chloroform and mixing adequately using a vortex, the samples were centrifuged for 20 minutes at 12,000 x g and 4°C. The precipitate (aqueous layer) obtained from this centrifugation was passed through an RNA recovery column of an RNeasy Midi Kit (Qiagen) and RNA was recovered by carrying out a washing procedure and an RNA elution procedure on the RNA recovery column in accordance with the protocol provided. The recovered RNA was quantified using a NanoDrop (NanoDrop).

Although the amount of RNA recovered from the stool sample (2) prepared using an ethanol solution serving as the removal solution S of the present invention was slightly less than the amount of RNA recovered from the stool sample (1) that was subjected to freezing treatment immediately after dispensing, an extremely large amount of RNA was able to be recovered in comparison with the stool sample (3) that was subjected to nucleic acid extraction immediately after dispensing. On the basis of these results, preparation of stool samples using the removal solution S of the present invention was determined to allow the obtaining of stool samples from which nucleic acid can be recovered extremely efficiently even if prepared at room temperature. In the case of a patient collecting stool at home as in the manner of routine health examinations and the like, although it is desirable that the stool sample be able to be prepared in the vicinity of room temperature, the removal solution S of the present invention is able to adequately respond to this requirement.

### [Reference Example 2]

1 g aliquots of stool collected from a healthy volunteer were dispensed into two 15 mL polypropylene tubes. 10 mL of a 70% ethanol solution were added to one of the tubes after dispensing followed by adequately dispersing the stool and allowing to stand for 1 hour at room temperature to obtain a stool sample (4). Subsequently, the ethanol in the stool sample (4) was removed followed by carrying out an extraction procedure. The contents of the remaining tube were suspended in 10 mL of phosphate buffer (pH 7.0) after dispensing the stool sample followed by centrifuging gently, recovering the supernatant and removing residue that was larger than cells. The recovered supernatant was centrifuged for 15 minutes at 3000 rpm, and the precipitate was recovered in the form of cells. 9 mL of 70% ethanol were added to the precipitate followed by mixing, centrifuging for 10 minutes at 3000 rpm to obtain a precipitate in the form of a stool sample (5), and subsequently carrying out an extraction procedure thereon. More specifically, 3 mL of a phenol mixture known as "Trizol" (Invitrogen) were added to each stool sample followed by adequately mixing for 30 seconds or more with a homogenizer. Subsequently, 3 mL of chloroform were added followed by adequately mixing using a vortex and centrifuging for 20 minutes at 12,000 x g and 4°C. The supernatant (aqueous layer) obtained by this centrifugation was passed through an RNA recovery column of an RNeasy Midi Kit (Qiagen) and RNA was.recovered by carrying out a washing procedure and an RNA elution procedure on the RNA recovery column in accordance with the protocol provided. The recovered RNA was quantified using a NanoDrop (NanoDrop).

cDNA was then synthesized using a portion of each RNA solution and a transcription reaction kit in the form of the ReverTra Ace qPCR RT Kit. Using this cDNA as template, 12.5 µL of 2xTaqMan PCR Master Mix (Perkin-Elmer Applied Biosystems) were added followed by the addition of a human GAPDH forward primer (SEQ ID NO. 1: 5'-GAAGGTGAAGGTCGGAGTC-3') and human GAPDH reverse primer (SEQ ID NO. 2: 5'-GAAGATGGTGATGGGATTTC-3') to respective final concentrations in the reaction solution of 900 nmol to prepare a PCR solution having a final volume of 25 µL. PCR analysis using SYBR Green and the ABI Prism 7700 (Perkin-Elmer Applied Biosystems) was then carried out on this PCR solution. PCR was carried out using a thermal cycle under the conditions of a denaturation cycle of 10 minutes at 95°C followed by 45 cycles consisting of 30 seconds at 95°C, 30 seconds at 55°C and 30 seconds at 72°C. Quantification was carried out based on the results of fluorescence intensity obtained by using a dilution series of known concentrations of a standard plasmid as template.
In the case of using RNA derived from sample (5) as template, a decrease in amplification efficiency of about 50% or more was observed in comparison with sample (4).
Namely, on the basis of these results, in the case of nucleic acid obtained using the nucleic acid recovery method of the present invention, in which collected stool is immediately immersed in the removal solution S and stored for a fixed period of time without preparing a suspension thereof, inhibitory substance A was determined to be removed from the recovered nucleic acid, recovery loss was low and nucleic acid amplification efficiency was determined to be favorable in comparison with nucleic acid obtained by using a nucleic acid recovery method in which the stool is first put into suspension.

Furthermore, although a PCR method that used DNA polymerase was used in the nucleic acid amplification reactions of the aforementioned examples and reference examples, RT-PCR, which is a nucleic acid amplification method that combines a reverse transcription reaction using RNA polymerase, or NASBA or TRC may also be included.

### INDUSTRIAL APPLICABILITY

According to the nucleic acid recovery method of the present invention, since substances that inhibit nucleic acid amplification reactions contained in a stool can be efficiently removed and nucleic acids of high purity can be efficiently recovered from a stool sample, the nucleic acid recovery method of the present invention can be used particularly in fields such as clinical testing, including routine health examinations using stool samples.

### BRIEF DESCRIPTION OF THE REFERENCE SYMBOLS

- 1: Container body
- 1a: Protrusion
- 2: Cover
- 3: Stool collection rod
- 3a: Cup
- S: Nucleic acid amplification reaction inhibitory substance removal solution
- 11: Container body
- 12: Cover
- 13: Stool collection rod
- 13a: Slot
- 13b: Movable cover
- 15: Pouch
- E: Stool
- 101: Stool sample processing apparatus
- 102: Centrifugal separation mechanism
- 103: Solution aspiration and discharge nozzle
- 104: Waste liquid recovery unit
- 105: Solution aspiration and discharge nozzle washing mechanism

### [SEQUENCE LISTINGS]

PCT International Patent Application No. PCT/JP2009/063163 sequence list

## Claims

1. A method for recovering nucleic acid from a stool sample at high purity, comprising:
(A) a step for eluting a nucleic acid amplification reaction inhibitory substance by preparing a stool sample by adding a collected stool to a solution for removing the nucleic acid amplification reaction inhibitory substance that has a water-soluble organic solvent as an active ingredient thereof, and storing the stool sample for a predetermined time,
(B) a step for recovering a stool-derived solid fraction by removing the solution for removing the nucleic acid amplification reaction inhibitory substances from the stool sample after step (A), and
(C) a step for recovering the nucleic acid from the stool-derived solid fraction recovered in step (B).

2. The method for recovering nucleic acid from a stool sample according to claim 1, wherein the stool sample is prepared by immediately adding the collected stool to the solution for removing the nucleic acid amplification reaction inhibitory substances without preparing a suspension.

3. The method for recovering nucleic acid from a stool sample according to claim 1 or 2, wherein the water-soluble organic solvent is a water-soluble alcohol and/or ketone.

4. The method for recovering nucleic acid from a stool sample according to any of claims 1 to 3, wherein a concentration of the water-soluble organic solvent is 30% or more.

5. The method for recovering nucleic acid from a stool sample according to claim 3 or 4, wherein the water-soluble organic solvent contains one or more members selected from the group consisting of ethanol, propanol and methanol as a water-soluble alcohol.

6. The method for recovering nucleic acid from a stool sample according to claim 1, wherein the water-soluble organic solvent used is ethanol.

7. The method for recovering nucleic acid from a stool sample according to claim 3 or 4, wherein the water-soluble organic solvent contains acetone and/or methyl ethyl ketone as a ketone.

8. The method for recovering nucleic acid from a stool sample according to any of claims 1 to 7, wherein a mixing ratio of the stool and the solution for removing the nucleic acid amplification reaction inhibitory substance is such that a volume of the solution for removing the nucleic acid amplification reaction inhibitory substance is preferably 1 or more when based on a value of 1 for stool volume.

9. The method for recovering nucleic acid from a stool sample according to any of claims 1 to 8, wherein removal of the solution for removing the nucleic acid amplification reaction inhibitory substance from the stool sample in the step (B) is carried out by centrifugal separation.

10. The method for recovering nucleic acid from a stool sample according to any of claims 1 to 9, wherein the amount of time during which the stool sample is stored in the step (A) is 1 hour or more.

11. The method for recovering nucleic acid from a stool sample according to any of claims 1 to 9, wherein the amount of time during which the stool sample is stored in the step (A) is 12 hours or more.

12. The method for recovering nucleic acid from a stool sample according to any of claims 1 to 9, wherein the amount of time during which the stool sample is stored in the step (A) is 24 hours or more.

13. The method for recovering nucleic acid from a stool sample according to any of claims 1 to 9, wherein the amount of time during which the stool sample is stored in the step (A) is 72 hours or more.

14. The method for recovering nucleic acid from a stool sample according to any of claims 1 to 13, wherein a temperature at which the stool sample is stored in the step (A) is 4°C or higher.

15. The method for recovering nucleic acid from a stool sample according to any of claims 1 to 13, wherein a temperature at which the stool sample is stored in the step (A) is 20°C or higher.

16. The method for recovering nucleic acid from a stool sample according to any of steps 1 to 15, wherein the solution for removing the nucleic acid amplification reaction inhibitory substance contains a detergent.

17. The method for recovering nucleic acid from a stool sample according to any of claims 1 to 16, wherein the solution for removing the nucleic acid amplification reaction inhibitory substance contains a colorant.

18. The method for recovering nucleic acid from a stool sample according to any of claims 1 to 17, wherein the step (C) is a step for simultaneously recovering the nucleic acid derived from indigenous intestinal bacteria and the nucleic acid derived from a creature other than indigenous intestinal bacteria from the stool-derived solid fraction recovered in step (B).

19. The method for recovering nucleic acid from a stool sample according to claim 18, wherein the creature other than indigenous intestinal bacteria is a mammalian cell.

20. The method for recovering nucleic acid from a stool sample according to any of claims 1 to 19, the step (C) comprises steps of:
(a) a step for denaturing a protein in the stool-derived solid fraction recovered in step (B), and eluting the nucleic acid from indigenous intestinal bacteria and a creature other than the indigenous intestinal bacteria present in the stool-derived solid fraction, and
(b) a step for recovering the nucleic acid eluted in the step (a).

21. The method for recovering nucleic acid from a stool sample according to claim 20, comprising steps of:
(c) a step for removing the protein denatured by the step (a) after the step (a) and before the step (b).

22. The method for recovering nucleic acid from a stool sample according to claim 20 or 21, wherein the protein denaturing in the step (a) is carried out using one or more members selected from the group consisting of a chaotropic salt, organic solvent and detergent.

23. The method for recovering nucleic acid from a stool sample according to claim 22, wherein the organic solvent is phenol.

24. The method for recovering nucleic acid from a stool sample according to any of claims 21 to 23, wherein the removal of denatured protein in the step (c) is carried out using chloroform.

25. The method for recovering nucleic acid from a stool sample according to any of claims 20 to 24, wherein the recovery of nucleic acid in the step (b) comprises steps of:
(b1) a step for adsorbing the nucleic acid eluted in the step (a) to an inorganic support, and
(b2) a step for eluting the nucleic acid adsorbed in the step (b1) from the inorganic support.

26. A nucleic acid recovered according to the method for recovering nucleic acid from a stool sample according to any of claims 1 to 25.

27. A nucleic acid analysis method in which a nucleic acid derived from a mammalian cell is analyzed using a nucleic acid recovered from a stool sample using the method for recovering nucleic acid from a stool sample according to any of claims 1 to 26.

28. The nucleic acid analysis method according to claim 27, wherein the mammalian cell is a gastrointestinal cell.

29. The nucleic acid analysis method according to claim 27, wherein the mammalian cell is an exfoliated colonocyte.

30. The nucleic acid analysis method according to any of claims 27 to 29, wherein the nucleic acid derived from the mammalian cell is a marker that indicates a neoplastic transformation.

31. The nucleic acid analysis method according to any of claims 27 to 29, wherein the nucleic acid derived from the mammalian cell is a marker that indicates an inflammatory gastrointestinal disease.

32. The nucleic acid analysis method according to any of claims 27 to 31, wherein the analysis is at least one member selected from the group consisting of mRNA expression analysis, K-ras gene mutation analysis, and DNA methylation analysis.

33. A stool sample processing apparatus, comprising: a solution removal mechanism that removes a solution for removing a nucleic acid amplification reaction inhibitory substance having a water-soluble organic solvent as an active ingredient thereof from a collected stool sample that has been immersed in the solution for removing the nucleic acid amplification reaction inhibitory substance and stored therein for a predetermined time.

34. The stool sample processing apparatus according to claim 33, wherein the solution removal mechanism comprises a centrifugal separation mechanism, a solution suction discharge mechanism, and a waste liquid recovery unit.

35. The stool sample processing apparatus according to claim 34, wherein the solution suction discharge mechanism is a solution suction discharge nozzle, and further comprises a mechanism that washes the solution suction discharge nozzle.

36. A solution for removing a nucleic acid amplification reaction inhibitory substance that is a solution used to prepare a stool sample for nucleic acid recovery, has a water-soluble organic solvent as an active ingredient thereof, and lowers the nucleic acid amplification reaction inhibitory substance present in the recovered nucleic acid.

37. The solution for removing a nucleic acid amplification reaction inhibitory substance according to claim 36, wherein the water-soluble organic solvent is a water-soluble alcohol and/or ketone.

38. The solution for removing a nucleic acid amplification reaction inhibitory substance according to claim 37, wherein a concentration of the water-soluble organic solvent is 30% or more.

39. A stool collection kit comprising the solution for a removing nucleic acid amplification reaction inhibitory substance according to any of claims 36 to 38, and a stool collection container that contains the solution for removing the nucleic acid amplification reaction inhibitory substance.


SEQUENCE LISTING

<110>  OLYMPUS CORPORATION, Ltd.
<120>  METHOD FOR RECOVERING NUCLEIC ACID FROM STOOL SAMPLE, NUCLEIC ACID
ANALYIS METHOD AND STOOL SAMPLE PROCESSING APPARATUS
<130>  09P00916
<160>  6
<170>  PatentIn version 3.1

<210>  1
<211>  19
<212>  DNA
<213>  Artificial Sequence
<220>
<223>  Description of Artificial Sequence: Human GAPDH forward primer.
<400>  1
gaaggtgaag gtcggagtc                                                    19

<210>  2
<211>  20
<212>  DNA
<213>  Artificial Sequence
<220>
<223>  Description of Artificial Sequence: Human GAPDH reverse primer.
<400>  2
gaagatggtg atgggatttc                                                   20

<210>  3
<211>  19
<212>  DNA
<213>  Artificial Sequence
<220>
<223>  Description of Artificial Sequence: 16S rRNA forward primer.
<400>  3
aggaggtgat ccaaccgca                                                    19

<210>  4
<211>  20
<212>  DNA
<213>  Artificial Sequence
<220>
<223>  Description of Artificial Sequence: 16S rRNA reverse primer.
<400>  4
aactggagga aggtggggat                                                   20

<210>  5
<211>  21
<212>  DNA
<213>  Artificial Sequence
<220>
<223>  Description of Artificial Sequence: K-ras codon12 GCT mutant forward
primer.
<400>  5
acttgtggta gttggagctg c                                                 21

<210>  6
<211>  24
<212>  DNA
<213>  Artificial Sequence
<220>
<223>  Description of Artificial Sequence: K-ras codon12 GCT mutant reverse
primer.
<400>  6
ctcatgaaaa tggtcagaga aacc                                              24